# EUROPÄISCHE PATENTSCHRIFT

(11) **EP 0 913 382 B1**
(45) Veröffentlichungstag und Bekanntmachung des Hinweises auf die Patenterteilung: **08.11.2000**
(21) Anmeldenummer: 98108750.5
(22) Anmeldetag: 14.05.1998
(51) Int. Cl.: C07C 45/29, C07B 33/00, C12P 7/00, C12P 7/24, C25B 3/02

(54) **Verfahren zur Herstellung von Aldehyden und Ketonen**
Process for the preparation of aldehydes and ketones
Procédé pour la préparation d'aldéhydes et de cétones

(30) Priorität: 06.06.1997 DE 19723961
(43) Veröffentlichungstag der Anmeldung: 06.05.1999
(73) Patentinhaber: Consortium für elektrochemische Industrie GmbH, 81379 München (DE)
(72) Erfinder: Fritz-Langhals, Elke, Dr., 85521 Ottobrunn (DE); Freudenreich, Johannes, Dr., 80331 München (DE); Candussio, Anton, Dr., 81825 München (DE); Hampp, Norbert, Prof. Dr., 35287 Amöneburg (DE)
(74) Vertreter: Potten, Holger

(56) Entgegenhaltungen:
- EP-A- 0 198 351
- EP-A- 0 775 684
- US-A- 3 322 833
- MASUI M ET AL: "N-hydroxyphthalimide as an effective mediator for the oxidation of alcohols by electrolysis" J. CHEM. SOC., CHEM. COMMUN. (JCCCAT,00224936);83; (8); PP.479-80, OSAKA UNIV.;FAC. PHARM. SCI.; OSAKA; 565; JAPAN (JP), XP002072181
- DATABASE WPI Section Ch, Week 8448 Derwent Publications Ltd., London, GB; Class E14, AN 84-298215 XP002072177 & JP 59 185 787 A (YOSHITOMI PHARM IND KK)
- DATABASE WPI Section Ch, Week 9022 Derwent Publications Ltd., London, GB; Class E19, AN 90-167582 XP002072178 & JP 02 107 790 A (OSAKA YUKI KAGAKU KOGYO KK)
- DATABASE WPI Section Ch, Week 9022 Derwent Publications Ltd., London, GB; Class E19, AN 90-167583 XP002072179 & JP 02 107 791 A (OSAKA YUKI KAGAKU KOGYO KK)
- DATABASE WPI Section Ch, Week 9038 Derwent Publications Ltd., London, GB; Class E19, AN 90-286026 XP002072180 & JP 02 200 653 A (OSAKA YUKIKAGAKU KO)
- DE NOOY A E J ET AL: "On the use of stable organic nitroxyl radicals for the oxidation of primary and secondary alcohols" SYNTHESIS (SYNTBF,00397881);96; (10); PP.1153-1174, TNO NUTRITION FOOD RESEARCH INSTITUTE;DEPARTMENT BIOCHEMISTRY; ZEIST; NL 3700; NETH. (NL), XP002072173
- EINHORN C ET AL: "Oxidation of organic substrates by molecular oxygen mediated by N-hydroxyphthalimide (NHPI) and acetaldehyde. " CHEM. COMMUN. (CAMBRIDGE); 97; (5); PP.447-448, UNIV. J. FOURIER;LAB. CHIM. BIOMIMET.; GRENOBLE; 38041; FR. (FR), XP002072174
- POTTHAST A ET AL: "A novel method for the conversion of benzyl alcohols to benzaldehydes by laccase-catalyzed oxidation" J. MOL. CATAL. A: CHEM. (JMCCF2,13811169);96; VOL.108 (1); PP.5-9, INSTITUT FUER HOLZ- UND PFLANZENCHEMIE, TECHNISCHE UNIVERSITAET DRESDEN, PIENNER STR. 23;THARANDT; D-01737; GERMANY (DE), XP002072175

## Beschreibung

Die Erfindung betrifft ein Verfahren zur Herstellung von Aldehyden und Ketonen.

Aldehyde und Ketone finden in der Organischen Chemie vielfach Verwendung. Sie sind z.B. wichtige Vorstufen bei der Synthese von Heterocyclen, von Duftstoffen und Farbstoffen.

Für die Herstellung von Aldehyden und Ketonen sind verschiedene Verfahren bekannt. Die direkte Einführung der Formyl- und der Acylgruppe in aromatische Systeme gelingt z.B. über elektrophile Substitutionsreaktionen, welche jedoch durch die Substitutionsregeln beschränkt sind. Aromatische Ketone können auch über metallorganische Reaktionen dargestellt werden. Nachteile, die insbesondere die Handhabung größerer Mengen erschweren, sind die Notwendigkeit wasserfreier Reaktionsmedien und die Verwendung toxischer Chemikalien wie Phosphoroxychlorid, Kohlenmonoxid, Zinkcyanid, Quecksilber- und Cadmiumorganyle.

Aldehyde und Ketone werden häufig über Oxidationsreaktionen ausgehend von den entsprechenden Alkoholen hergestellt. Eine Übersicht bietet die in Houben-Weyl-Müller, Bd. 7/2a, S. 699ff und Bd. E3, S. 265 ff, 1983, referierte Literatur. Sie werden meist in nichtwäßrigem Medium durchgeführt und benötigen oft teure und/oder toxische Reagenzien, die aufgrund ihrer schwierigen Handhabbarkeit ungeeignet sind für Synthesen in größerem Maßstab. Aromatische Aldehyde und Ketone können aus Hydroxyalkylaromaten mit stabilen Nitroxylradikalen, z.B. TEMPO, und einem Oxidationsmittel hergestellt werden (A. E. J. de Nooy, A. C. Besemer und H. V. Bekkum, Synthesis **1996**, 1153). Oxidierendes Agens hierbei ist ein Oxoammoniumion, welches sich durch Zusatz eines Oxidationsmittels bildet. Nachteil dieser Methode ist einerseits der hohe Preis der benötigten Reagenzien, andererseits ist ein nichtwäßriges Medium erforderlich, da sonst eine Weiteroxidation des gebildeten Aldehyds zur Carbonsäure erfolgt. Die Bildung aromatischer Aldehyde aus den entsprechenden Hydroxymethylaromaten in Gegenwart des Enzyms Laccase und ABTS
(2,2'-Azinobis(3-ethylbenzthiazolin-6-sulfonsäure) wurde von Potthast (Potthast et al., J. Molec. Catal. A: Chemical **1996**, 108, 5 und Synth. Commun. **1996**, 26, 315) beobachtet. Der hohe Preis von ABTS schließt jedoch eine Verwendbarkeit der Methode in technischem Maßstab aus. Ein weiterer Nachteil ist die geringe Selektivität in Gegenwart von Methyl- und Hydroxymethylgruppen.

I. Chem.Soc., Chem. Commun.: 83;(8); S. 479-480 offenbart eine Methode zur elektrochemischen Oxidation von primären und sekundären Arylmethanolen unter zur Hilfenahme von N-Hydroxyphthalimid als Mediator zu den korrespondierenden Aldehyden bzw. Ketonen

Es besteht ein Bedarf an einem kostengünstigen und selektiven Verfahren, welches den stets steigenden Anforderungen in der Technik genügt und mit welchen auch empfindliche Aldehyde und Ketone in großem Maßstab dargestellt werden können.

Die Erfindung betrifft ein Verfahren gemäß Anspruch 1. Die Erfindung betrifft ferner ein Verfahren gemäß Anspruch 7.

Bevorzugt bedeutet Y¹ aromatischer oder heteroaromatischer Ring oder Ringsystem mit bis zu 6 Ringen und mit bis zu 20 C-Atomen, dessen Ringglieder durch O-, S- oder N-Atome ersetzt sein können oder Anthrachinonylrest,wobei der aromatische oder heteroaromatische Rest Y¹ ein- bis sechsfach substituiert sein kann,
wobei die Substituenten gleich oder verschieden sein können und die Bedeutung haben von OH, eines linearen, verzweigten oder cyclischen C₃-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe, deren Phenylreste ein- bis fünffach substituiert sein können, wobei die Substituenten gleich oder verschieden sein können und die Bedeutung haben von OH, eines linearen, verzweigten oder cyclischen C₁-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe oder
gegebenenfalls ein- bis dreifach substituierter Vinylrest, oder gegebenenfalls substituierter Ethinylrest,
worin die Substituenten gleich oder verschieden sein können und Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können, oder ein Anthrachinonylrest sein können, oder worin die Vinylgruppe Bestandteil eines Ringes oder Ringsystems ist.
Bevorzugt bedeutet Y² Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können, oder Anthrachinonylrest oder gegebenenfalls ein- bis dreifach substituierter Vinylrest, oder gegebenenfalls substituierter Ethinylrest,
worin die Substituenten gleich oder verschieden sein können und Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können, oder ein Anthrachinonylrest sein können, oder worin die Vinylgruppe Bestandteil eines Ringes oder Ringsystems ist.

Gleichermaßen bevorzugt sind Verbindungen, bei welchen die Reste Y¹ und Y² über eine Methylengruppe oder eine Ethergruppe oder eine gegebenenfalls mit einem linearen,verzweigten oder cyclischen C₁-C₁₂-Alkylrest substituierte Aminogruppe verknüpft sind.

Besonders bevorzugt bedeutet Y¹ 5-, 6- oder 7-gliedriger aromatischer oder heteroaromatischer Ring, der mit ein oder zwei weiteren aromatischen Ringen annelliert sein kann und bei dem ein bis vier C-Atome durch O-, S- oder N-Atome ersetzt sein können, oder ein Anthrachinonylrest,
wobei Y¹ ein- bis vierfach substituiert sein kann,
wobei die Substituenten gleich oder verschieden sein können und die Bedeutung haben von OH, eines linearen, verzweigten oder cyclischen C₁-C₆-Alkylrestes, wobei benachbarte Alkylgruppen über eine Methylengruppe einen 5- oder 6-gliedrigen Ring bilden können, oder eines linearen oder verzweigten C₁-C₆-Oxyalkyl- oder -Thioalkylrests, wobei benachbarte Substituenten über eine Methylengruppe einen 5-oder 6-gliedrigen Ring bilden können, einer H₂N-, oder einer linearen oder verzweigten C₁-C₆-N-Alkylamino-, einer linearen oder verzweigten C₁-C₃-N,N-Dialkylaminogruppe, NC-, O₂N-, Halogen, HOOC-, HO₃S-, OHC-, H₂N-COO-, H₂N-CO-, H₂N-CO-NH-Rest, oder einer linearen, verzweigten oder cyclischen C₁-C₆-OCO-, C₁-C₆-COO-, C₁-C₆-CO-, C₁-C₆-NHCO-, C₁-C₆-NHCONH-, (C₁-C₆)₂NCO-, C₁-C₆-CONH-, oder einer linearen oder verzweigten C₁-C₆-OSO₂-, C₁-C₆-NH-SO₂- oder (C₁-C₃)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe,
wobei die Phenylreste ein- bis dreifach substituiert sein können, wobei die Substituenten gleich oder verschieden sein können und die Bedeutung haben von OH, eines linearen, verzweigten oder cyclischen C₁-C₆-Alkylrestes, wobei benachbarte Alkylgruppen über eine Methylengruppe einen 5- oder 6-gliedrigen Ring bilden können, oder eines linearen oder verzweigten C₁-C₆-Oxyalkyl- oder -Thioalkylrests, wobei benachbarte Substituenten über eine Methylengruppe einen 5-oder 6-gliedrigen Ring bilden können, einer H₂N-, oder einer linearen oder verzweigten C₁-C₆-N-Alkylamino-, einer linearen oder verzweigten C₁-C₃-N,N-Dialkylaminogruppe, NC-, O₂N-, Halogen, HOOC-, HO₃S-, OHC-, H₂N-COO-, H₂N-CO-, H₂N-CO-NH-Rest, oder einer linearen, verzweigten oder cyclischen C₁-C₆-OCO-, C₁-C₆-COO-, C₁-C₆-CO-, C₁-C₆-NHCO-, C₁-C₆-NHCONH-, (C₁-C₆)₂NCO-, C₁-C₆-CONH-, oder einer linearen oder verzweigten C₁-C₆-OSO₂-, C₁-C₆-NH-SO₂- oder (C₁-C₃)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder
Phenyl-NH-SO₂-gruppe oder
einen gegebenenfalls ein- bis dreifach substituierten Vinylrest, oder einen gegebenenfalls substituierten Ethinylrest bedeutet, worin die Substituenten gleich oder verschieden sein können und die Bedeutung haben von Wasserstoff, ein linearer, verzweigter oder cyclischer C₁-C₆-Alkylrest, bei dem eine oder zwei Methylengruppen einzeln durch -CHOH, -CO, O-, S-, NH- oder durch einen linearen oder verzweigten C₁-C₆-N-Alkylamin-Rest ersetzt sein können, oder aromatischer oder heteroaromatischer Ring oder Ringsystem mit bis zu 6 Ringen und mit bis zu 20 C-Atomen, dessen Ringglieder durch O-, S- oder N-Atome ersetzt sein können oder ein Anthrachinonylrest.

Besonders bevorzugt bedeutet Y² Wasserstoff, linearer, verzweigter oder cyclischer C₁-C₆-Alkylrest, bei dem eine oder zwei Methylengruppen einzeln durch -CHOH, -CO, O-, S-, NH- oder durch einen linearen oder verzweigten C₁-C₆-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können, oder Anthrachinonylrest oder einen gegebenenfalls ein- bis dreifach substituierten Vinylrest, oder einen gegebenenfalls substituierten Ethinylrest, worin die Substituenten gleich oder verschieden sein können und die Bedeutung haben von Wasserstoff, ein linearer, verzweigter oder cyclischer C₁-C₆-Alkylrest, bei dem eine oder zwei Methylengruppen einzeln durch -CHOH, -CO, O-, S-, NH- oder durch einen linearen oder verzweigten C₁-C₆-N-Alkylamin-Rest ersetzt sein können, oder aromatischer oder heteroaromatischer Ring oder Ringsystem mit bis zu 6 Ringen und mit bis zu 20 C-Atomen, dessen Ringglieder durch O-, S- oder N-Atome ersetzt sein können.

Gleichermaßen besonders bevorzugt sind Verbindungen, bei welchen die Reste Y¹ und Y² über eine Methylengruppe oder eine Ethergruppe oder eine gegebenenfalls mit einem linearen, verzweigten oder cyclischen C₁-C₆-Alkylrest substituierte Aminogruppe verknüpft sind.

Ganz besonders bevorzugt bedeutet Y¹ Phenyl, Naphthyl, Anthryl, Phenanthryl, Azulenyl, Anthrachinonyl, Furyl, Pyrrolyl, Thienyl, Benzofuranyl, Isobenzofuranyl, Benzothiyl, Isobenzothienyl, Indolyl, Isoindolyl, Indolizinyl, Pyrazolyl, Imidazolyl, Oxazolyl, Thiazolyl, Isoxazolyl, Isothiazolyl, Indazolyl, Carbazolyl, Benzotriazolyl, Purinyl, Pyridyl, Pyridazinyl, Pyrimidyl, Pyrazinyl, Chinolinyl, Isochinolinyl, Chinoxalinyl, Chinazolinyl, Cinnolinyl, Phenanthridinyl, Acridinyl, 1,10-Phenanthrolinyl, Phenazinyl, Phenothiazinyl, Phenoxazinyl-Rest, wobei Y¹ ein- bis dreifach substituiert sein kann, wobei die Substituenten gleich oder verschieden sein können und die Bedeutung haben von OH, eines linearen C₁-C₆-Alkylrestes, wobei benachbarte Alkylgruppen über eine Methylengruppe einen 5- oder 6-gliedrigen Ring bilden können, oder eines linearen C₁-C₆-Oxyalkylrests, wobei benachbarte Substituenten über eine Methylengruppe einen 5-oder 6-gliedrigen Ring bilden können, einer H₂N-, oder einer linearen C₁-C₆-N-Alkylamino-, einer linearen C₁-C₃-N,N-Dialkylaminogruppe, NC-, O₂N-, Halogen, HOOC-, HO₃S-, OHC-, H₂N-COO-, H₂N-CO-, H₂N-CO-NH-Rest, oder einer linearen C₁-C₄OCO-, C₁-C₄-COO-, C₁-C₄-CO-, C₁-C₄-NHCO-, (C₁-C₄)₂NCO-, C₁-C₄-CONH-, C₁-C₄-NHCONH-, oder C₁-C₄-OSO₂-, C₁-C₄-NH-SO₂- oder (C₁-C₃)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe, wobei die Phenylreste ein bis dreifach substituiert sein können,wobei die Substituenten gleich oder verschieden sein können und die Bedeutung haben von OH, eines linearen C₁-C₆-Alkylrestes, wobei benachbarte Alkylgruppen über eine Methylengruppe einen 5- oder 6-gliedrigen Ring bilden können, oder eines linearen C₁-C₆-Oxyalkylrests, wobei benachbarte Substituenten über eine Methylengruppe einen 5-oder 6-gliedrigen Ring bilden können, einer H₂N-, oder einer linearen C₁-C₆-N-Alkylamino-, einer linearen C₁-C₃-N,N-Dialkylaminogruppe, NC-, O₂N-, Halogen, HOOC-, HO₃S-, OHC-, H₂N-COO-, H₂N-CO-, H₂N-CO-NH-, oder einer linearen C₁-C₄OCO-, C₁-C₄-COO-, C₁-C₄-CO-, C₁-C₄-NHCO-, (C₁-C₄)₂NCO-, C₁-C₄-CONH-, C₁-C₄-NHCONH-, oder C₁-C₄-OSO₂-, C₁-C₄-NH-SO₂- oder (C₁-C₃)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe oder Y¹ gegebenenfalls ein- bis dreifach substituierter Vinylrest, oder gegebenenfalls substituierter Ethinylrest bedeutet, worin die Substituenten gleich oder verschieden sein können und die Bedeutung haben von Wasserstoff, linearer C₁-C₆-Alkylrest, bei dem eine oder zwei Methylengruppen einzeln durch -CHOH, -CO, O-, S-, NH- oder durch einen linearen oder verzweigten C₁-C₆-N-Alkylamin-Rest ersetzt sein können, oder aromatischer oder heteroaromatischer Ring oder Ringsystem mit bis zu 6 Ringen und mit bis zu 20 C-Atomen, dessen Ringglieder durch O-, S- oder N-Atome ersetzt sein können oder Anthrachinonylrest, oder worin die Vinylgruppe Bestandteil eines 5- oder 6-gliedrigen Ringes oder eines Ringsystems ist,
und Y² Wasserstoff, linearer C₁-C₆-Alkylrest, bei dem eine oder zwei Methylengruppen einzeln durch -CHOH, -CO, O-, S-, NH- oder durch einen linearen oder verzweigten C₁-C₆-N-Alkylamin-Rest ersetzt sein können, oder aromatischer oder heteroaromatischer Ring oder Ringsystem mit bis zu 6 Ringen und mit bis zu 20 C-Atomen, dessen Ringglieder durch O-, S- oder N-Atome ersetzt sein können oder Anthrachinonylrest oder gegebenenfalls ein- bis dreifach substituierter Vinylrest, oder gegebenenfalls substituierter Ethinylrest, worin die Substituenten gleich oder verschieden sein können und die Bedeutung haben von Wasserstoff, linearer C₁-C₆-Alkylrest, bei dem eine oder zwei Methylengruppen einzeln durch -CHOH, -CO, O-, S-, NH- oder durch einen linearen oder verzweigten C₁-C₆-N-Alkylamin-Rest ersetzt sein können, oder aromatischer oder heteroaromatischer Ring oder Ringsystem mit bis zu 6 Ringen und mit bis zu 20 C-Atomen, dessen Ringglieder durch O-, S- oder N-Atome ersetzt sein können oder Anthrachinonylrest, oder worin die Vinylgruppe Bestandteil eines 5- oder 6-gliedrigen Ringes oder eines Ringsystems ist.

Die folgenden Ausführungen vom Mediator beziehen sich ausschließlich auf Mediatoren im Verfahren gemäß Anspruch 1.

Als Mediator wird vorzugsweise mindestens eine Verbindung ausgewählt aus der Gruppe der aliphatischen, cycloaliphatischen, heterocyclischen oder aromatischen Verbindungen, die mindestens eine N-Hxdroxy-, Oxim-, Nitroso-, N-Oxyl- oder N-Oxi Funktion enthält.

Beispiele für solche Verbindungen sind die im folgenden genannten Verbindungen der Formel I, II, III oder IV, wobei die Verbindungen der Formeln II, III und IV bevorzugt und die Verbindungen der Formeln III und IV besonders bevorzugt sind.

Verbindungen der allgemeinen Formel I sind: 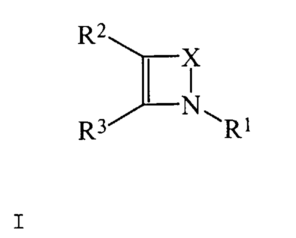 wobei X für eine der folgenden Gruppen steht:
(-N=N-), (-N=CR⁴-)ₚ, (-CR⁴=N-)ₚ, (-CR⁵=CR⁶)ₚ 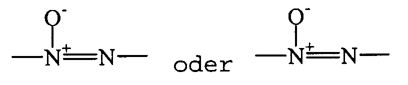 und p gleich 1 oder 2 ist,
wobei die Reste R¹ bis R⁶ gleich oder verschieden sein können und unabhängig voneinander eine der folgenden Gruppen darstellen können: Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-alkyl, Phenyl, Sulfono, Ester und Salze davon, Sulfamoyl, Carbamoyl, Phospho, Phosphono, Phosphonooxy und deren Salze und Ester und wobei die Amino-, Carbamoyl- und Sulfamoyl-Gruppen der Reste R¹ bis R⁶ weiterhin unsubstituiert oder ein- oder zweifach mit Hydroxy, C₁-C₃-Alkyl, C₁-C₃-Alkoxy substituiert sein können,
und wobei die Reste R² und R³ eine gemeinsame Gruppe -A- bilden können und -A- dabei eine der folgenden Gruppen repräsentiert: (-CR⁷=CR⁸-CR⁹=CR¹⁰-) oder (-CR¹⁰=CR⁹-CR⁸=CR⁷-).

Die Reste R⁷ bis R¹⁰ können gleich oder ungleich sein und unabhängig voneinander eine der folgenden Gruppen darstellen: Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-Alkyl, Phenyl, Sulfono, Ester und Salze davon, Sulfamoyl, Carbamoyl, Phospho, Phosphono, Phosphonooxy und deren Salze und Ester und wobei die Amino-, Carbamoyl- und Sulfamoyl-Gruppen der Reste R⁷ bis R¹⁰ weiterhin unsubstituiert oder ein- oder zweifach mit Hydroxy, C₁₋C₃-Alkyl, C₁-C₃-Alkoxy substituiert sein können und wobei die C₁-C₁₂-Alkyl-, C₁-C₆-Alkyloxy-, Carbonyl-C₁-C₆-alkyl-, Phenyl-, Aryl-Gruppen der Reste R⁷ bis R¹⁰ unsubstituiert oder weiterhin ein- oder mehrfach mit dem Rest R¹¹ substituiert sein können und wobei der Rest R¹¹ eine der folgenden Gruppen darstellen kann: Wasserstoff, Halogen, Hydroxy, Formyl, Carboxy sowie deren Salze und Ester, Amino, Nitro, C₁-C₁₂-Alkyl, C₁-C₆-Alkyloxy, Carbonyl-C₁-C₆-Alkyl, Phenyl, Aryl, sowie deren Ester und Salze und wobei die Carbamoyl, Sulfamoyl, Amino-Gruppen des Restes R¹¹ unsubstituiert oder weiterhin ein- oder zweifach mit dem Rest R¹² substituiert sein können und wobei der Rest R¹² eine der folgenden Gruppen darstellen kann: Wasserstoff, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl.

Beispiele für die genannten Verbindungen sind:
1-Hydroxy-1,2,3-triazol-4,5-dicarbonsäure
1-Phenyl-1H-1,2,3-triazol-3-oxid
5-chlor-1-phenyl-1H-1,2,3-triazol-3-oxid
5-Methyl-1-phenyl-1H-1,2,3-triazol-3-oxid
4-(2,2-Dimethylpropanoyl)-1-hydroxy-1H-1,2,3-triazol
4-Hydroxy-2-phenyl-2H-1,2,3-triazol-1-oxid
2,4,5-Triphenyl-2H-1,2,3-triazol-1-oxid
1-Benzyl-1H-1,2,3-triazol-3-oxid
1-Benzyl-4-chlor-1H-1,2,3-triazol-3-oxid
1-Benzyl-4-brom-1H-1,2,3-triazol-3-oxid
1-Benzyl-4-methoxy-1H-1,2,3-triazol-3-oxid

Verbindungen der allgemeinen Formel II sind: 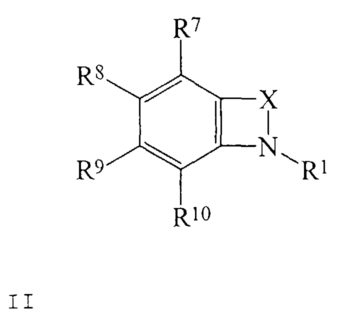 wobei X für eine der folgenden Gruppen steht:
(-N=N-), (-N=CR⁴-)ₚ, (-CR⁴=N-)ₚ, (-CR⁵=CR⁶)ₚ 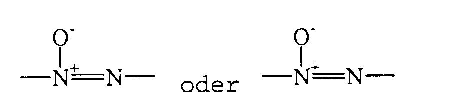 und p gleich 1 oder 2 ist.

Die Reste R¹ und R⁴ bis R¹⁰ können gleich oder ungleich sein und unabhängig voneinander eine der folgenden Gruppen darstellen:
Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-alkyl, Phenyl, Aryl, Sulfono, Ester und Salze davon, Sulfamoyl, Carbamoyl, Phospho, Phosphono, Phosphonooxy und deren Salze und Ester und wobei die Amino-, Carbamoyl- und Sulfamoyl-Gruppen der Reste R¹ und R⁴ bis R¹⁰ weiterhin unsubstituiert oder ein oder zweifach mit Hydroxy, C₁-C₃-Alkyl, C₁-C₃-Alkoxy substituiert sein können
und wobei die C₁-C₁₂-Alkyl-, C₁-C₆-Alkyloxy-, Carbonyl-C₁₋C₆-Alkyl-, Phenyl-, Aryl-, Aryl-C₁-C₆-alkyl-Gruppen der Reste R¹ und R⁴ bis R¹⁰ unsubstituiert oder weiterhin ein- oder mehrfach mit dem Rest R¹² substituiert sein können und wobei der Rest R¹² eine der folgenden Gruppen darstellen kann: Wasserstoff, Halogen, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl, Sulfono, Sulfeno, Sulfino und deren Ester und Salze
und wobei die Carbamoyl-, Sulfamoyl-, Amino-Gruppen des Restes R¹² unsubstituiert oder weiterhin ein- oder zweifach mit dem Rest R¹³ substituiert sein können und wobei der Rest R¹³ eine der folgenden Gruppen darstellen kann: Wasserstoff, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl.

Beispiele für die genannten Verbindungen sind:

### 1-Hydroxy-benzimidazole

1-Hydroxybenzimidazol-2-carbonsäure
1-Hydroxybenzimidazol
2-Methyl-1-hydroxybenzimidazol
2-Phenyl-1-hydroxybenzimidazol

### 1-Hydroxyindole

2-Phenyl-1-hydroxyindol

Substanzen der allgemeinen Formel III sind: 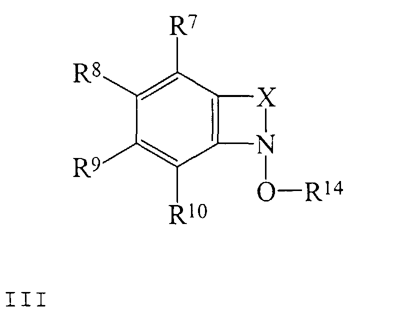 wobei X für eine der folgenden Gruppen steht:
(-N=N-), (-N=CR⁴-)ₘ, (-CR⁴=N-)ₘ, (-CR⁵=CR⁶-)ₘ 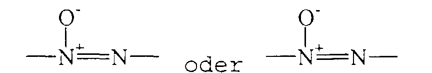 und m gleich 1 oder 2 ist.

Für die Reste R⁷ bis R¹⁰ und R⁴ bis R⁶ gilt das oben gesagte.

R¹⁴ kann sein: Wasserstoff, C₁-C₁₀-alkyl, C₁-C₁₀-alkylcarbonyl, deren C₁-C₁₀-alkyl und C₁-C₁₀-alkylcarbonyl unsubstituiert oder mit einem Rest R¹⁵ ein- oder mehrfach substituiert sein können wobei R¹⁵ eine der folgenden Gruppen darstellen kann: Wasserstoff, Halogen, Hydroxy-, Formyl-, Carboxy- sowie Salze und Ester davon, Amino-, Nitro-, C₁-C₁₂-Alkyl, C₁-C₆-Alkyloxy, Carbonyl-C₁-C₆-Alkyl-, Phenyl-, Sulfono-, deren Ester und Salze, Sulfamoyl-, Carbamoyl-, Phospho-, Phosphono-, Phosphonooxy- und deren Salze und Ester,
wobei die Amino-, Carbamoyl- und Sulfamoyl-Gruppen des Restes R¹⁵ weiterhin unsubstituiert oder ein- oder zweifach mit Hydroxy-, C₁-C₃-Alkyl-, C₁-C₃-Alkoxy- substituiert sein können.

Von den Substanzen der Formel III sind insbesondere Derivate des 1-Hydroxybenzotriazols und des tautomeren Benzotriazol-1-oxides sowie deren Ester und Salze bevorzugt (Verbindungen der Formel IV)

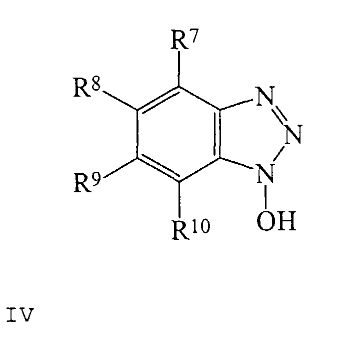

Die Reste R⁷ bis R¹⁰ können gleich oder verschieden sein und unabhängig voneinander eine der folgenden Gruppen darstellen: Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-alkyl, Phenyl, Sulfono, Ester und Salze davon, Sulfamoyl, Carbamoyl, Phospho, Phosphono, Phosphonooxy und deren Salze und Ester und wobei die Amino-, Carbamoyl- und Sulfamoyl-Gruppen der Reste R⁷ bis R¹⁰ weiterhin unsubstitiert oder ein- oder zweifach mit Hydroxy, C₁₋C₃-Alkyl, C₁-C₃-Alkoxy substituiert sein können und wobei die C₁-C₁₂-Alkyl-, C₁-C₆-Alkyloxy-, Carbonyl-C₁-C₆-alkyl-, Phenyl-, Aryl-Gruppen der Reste R⁷ bis R¹⁰ unsubstituiert oder weiterhin ein oder mehrfach mit dem Rest R¹⁶ substituiert sein können und wobei der Rest R¹⁶ eine der folgenden Gruppen darstellen kann: Wasserstoff, Halogen, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl, Sulfono, Sulfeno, Sulfino sowie deren Ester und Salze und wobei die Carbamoyl-, Sulfamoyl-, amino-Gruppen des Restes R¹⁶ unsubstituiert oder weiterhin ein- oder zweifach mit dem Rest R¹⁷ substituiert sein können und wobei der Rest R¹⁷ eine der folgenden Gruppen darstellen kann: Wasserstoff, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl.

Beispiele für die genannten Verbindungen sind:

### 1H-Hydroxybenzotriazole

1-Hydroxybenzotriazol
1-Hydroxybenzotriazol, Natriumsalz.
1-Hydroxybenzotriazol, Kaliumsalz
1-Hydroxybenzotriazol, Lithiumsalz
1-Hydroxybenzotriazol, Ammoniumsalz
1-Hydroxybenzotriazol, Calciumsalz
1-Hydroxybenzotriazol, Magnesiumsalz
1-Hydroxybenzotriazol-6-sulfonsäure
1-Hydroxybenzotriazol-6-sulfonsäure, Mononatriumsalz
1-Hydroxybenzotriazol-6-carbonsäure
1-Hydroxybenzotriazol-6-N-phenylcarboxamid
5-Ethoxy-6-nitro-1-hydroxybenzotriazol
4-Ethyl-7-methyl-6-nitro-1-hydroxybenzotriazol
2,3-Bis-(4-ethoxy-phenyl)-4,6-dinitro-2,3-dihydro-1-hydroxybenzotriazol
2,3-Bis-(2-brom-4-methyl-phenyl)-4,6-dinitro-2,3-dihydro-1-hydroxybenzotriazol
2,3-Bis-(4-brom-phenyl)-4,6-dinitro-2,3-dihydro-1-hydroxybenzotriazol
2,3-Bis-(4-carboxy-phenyl)-4,6-dinitro-2,3-dihydro-1-hydroxy-benzotriazol
4,6-Bis-(trifluormethyl)-1-hydroxybenzotriazol
5-Brom-1-hydroxybenzotriazol
6-Brom-1-hydroxybenzotriazol
4-Brom-7-methyl-1-hydroxybenzotriazol
5-Brom-7-methyl-6-nitro-1-hydroxybenzotriazol
4-Brom-6-nitro-1-hydroxybenzotriazol
6-Brom-4-nitro-1-hydroxybenzotriazol
4-Chlor-1-hydroxybenzotriazol
5-Chlor-1-hydroxybenzotriazol
6-Chlor-1-hydroxybenzotriazol
6-Chlor-5-isopropyl-1-hydroxybenzotriazol
5-Chlor-6-methyl-1-hydroxybenzotriazol
6-Chlor-5-methyl-1-hydroxybenzotriazol
4-Chlor-7-methyl-6-nitro-1-hydroxybenzotriazol
4-Chlor-5-methyl-1-hydroxybenzotriazol
5-chlor-4-methyl-1-hydroxybenzotriazol
4-Chlor-6-nitro-1-hydroxybenzotriazol
6 -Chlor-4-nitro-1-hydroxybenzotriazol
7-Chlor-1-hydroxybenzotriazol
6-Diacetylamino-1-hydroxybenzotriazol
2,3-Dibenzyl-4,6-dinitro-2,3-dihydro-1-hydroxybenzotriazol
4,6-Dibrom-1-hydroxybenzotriazol
4,6-Dichlor-1-hydroxybenzotriazol
5,6-Dichlor-1-hydroxybenzotriazol
4,5-Dichlor-1-hydroxybenzotriazol
4,7-Dichlor-1-hydroxybenzotriazol
5,7-Dichlor-6-nitro-1-hydroxybenzotriazol
5,6-Dimethoxy-1-hydroxybenzotriazol
2,3-Di-[2]naphthyl-4,6-dinitro-2,3-dihydro-1-hydroxybenzotriazol
4,6-Dinitro-1-hydroxybenzotriazol
4,6-Dinitro-2,3-diphenyl-2,3-dihydro-1-hydroxybenzotriazol
4,6-Dinitro-2,3-di-p-tolyl-2,3-dihydro-1-hydroxybenzotriazol
5-Hydrazino-7-methyl-4-nitro-1-hydroxybenzotriazol
5,6-Dimethyl-1-hydroxybenzotriazol
4-Methyl-1-hydroxybenzotriazol
5-Methyl-1-hydroxybenzotriazol
6-Methyl-1-hydroxybenzotriazol
5-(1-Methylethyl)-1-hydroxybenzotriazol
4-Methyl-6-nitro-1-hydroxybenzotriazol
6-Methyl-4-nitro-1-hydroxybenzotriazol
5-Methoxy-1-hydroxybenzotriazol
6-Methoxy-1-hydroxybenzotriazol
7-Methyl-6-nitro-1-hydroxybenzotriazol
4-Nitro-1-hydroxybenzotriazol
6-Nitro-1-hydroxybenzotriazol
6-Nitro-4-phenyl-1-hydroxybenzotriazol
5-Phenylmethyl-1-hydroxybenzotriazol
4-Trifluormethyl-1-hydroxybenzotriazol
5-Trifluormethyl-1-hydroxybenzotriazol
6-Trifluormethyl-1-hydroxybenzotriazol
4,5,6,7-Tetrachlor-1-hydroxybenzotriazol
4,5,6,7-Tetrafluor-1-hydroxybenzotriazol
6-Tetrafluorethyl-1-hydroxybenzotriazol
4,5,6-Trichlor-1-hydroxybenzotriazol
4,6,7-Trichlor-1-hydroxybenzotriazol
6-Sulfamido-1-hydroxybenzotriazol
6-N,N-Diethyl-sulfamido-1-hydroxybenzotriazol
6-N-Methylsulfamido-1-hydroxybenzotriazol
6-(1H-1,2,4-triazol-1-ylmethyl)-1-hydroxybenzotriazol
6-(5,6,7,8-tetrahydroimidazo-[1,5-a]-pyridin-5-yl)-1-hydroxy-benzotriazol
6-(Phenyl-1H-1,2,4-triazol-1-ylmethyl)-1-hydroxybenzotriazol
6-[(5-methyl-1H-imidazo-1-yl)-phenylmethyl]-1-hydroxybenzotriazol
6-(4-methyl-1H-imidazo-1-yl)-phenylmethyl]-1-hydroxybenzotriazol
6-[(2-methyl-1H-imidazo-1-yl)-phenylmethyl]-1-hydroxybenzotriazol
6-(1H-Imidazol-1-yl-phenylmethyl)-1-hydroxybenzotriazol
5-(1H-Imidazol-1-yl-phenylmethyl)-1-hydroxybenzotriazol
6-[1-(1H-Imidazol-1-yl)-ethyl]-1-hydroxybenzotriazol-monohydrochlorid

### 3H-Benzotriazol-1-Oxide

### 3H-Benzotriazol-1-oxid

6-Acetyl-3H-benzotriazol-1-oxid
5-Ethoxy-6-nitro-3H-benzotriazol-1-oxid
4-Ethyl-7-methyl-6-nitro-3H-benzotriazol-1-oxid
6-Amino-3,5-dimethyl-3H-benzotriazol-1-oxid
6-Amino-3-methyl-3H-benzotriazol-1-oxid
5-Brom-3H-benzotriazol-1-oxid
6-Brom-3H-benzotriazol-1-oxid
4-Brom-7-methyl-3H-benzotriazol-1-oxid
5-Brom-4-chlor-6-nitro-3H-benzotriazol-1-oxid
4-Brom-6-nitro-3H-benzotriazol-1-oxid
6-Brom-4-nitro-3H-benzotriazol-1-oxid
5-Chlor-3H-benzotriazol-1-oxid
6-Chlor-3H-benzotriazol-1-oxid
4-Chlor-6-nitro-3H-benzotriazol-1-oxid
4,6-Dibrom-3H-benzotriazol-1-oxid
4,6-Dibrom-3-methyl-3H-benzotriazol-1-oxid
4,6-Dichlor-3H-benzotriazol-1-oxid
4,7-Dichlor-3H-benzotriazol-1-oxid
5,6-Dichlor-3H-benzotriazol-1-oxid
4,6-Dichlor-3-methyl-3H-benzotriazol-1-oxid
5,7-Dichlor-6-nitro-3H-benzotriazol-1-oxid
3,6-Dimethyl-6-nitro-3H-benzotriazol-1-oxid
3,5-Dimethyl-6-nitro-3H-benzotriazol-1-oxid
3-Methyl-3H-benzotriazol-1-oxid
5-Methyl-3H-benzotriazol-1-oxid
6-Methyl-3H-benzotriazol-1-oxid
6-Methyl-4-nitro-3H-benzotriazol-1-oxid
7-Methyl-6-nitro-3H-benzotriazol-1-oxid
5-Chlor-6-nitro-3H-benzotriazol-1-oxid

### 2H-Benzotriazol-1-oxide

2-(4-Acetoxy-phenyl)-2H-benzotriazol-1-oxid
6-Acetylamino-2-phenyl-2H-benzotriazol-1-oxid
2-(4-Ethyl-phenyl)-4,6-dinitro-2H-benzotriazol-1-oxid
2-(3-Aminophenyl)-2H-benzotriazol-1-oxid
2-(4-Aminophenyl)-2H-benzotriazol-1-oxid
6-Amino-2-phenyl-2H-benzotriazol-1-oxid
5-Brom-4-chlor-6-nitro-2-phenyl-2H-benzotriazol-1-oxid
2-(4-Bromphenyl)-2H-benzotriazol-1-oxid
5-Brom-2-phenyl-2H-benzotriazol-1-oxid
6-Brom-2-phenyl-2H-benzotriazol-1-oxid
2-(4-Bromphenyl)-4,6-dinitro-2H-benzotriazol-1-oxid
2-(4-Bromphenyl)-6-nitro-2H-benzotriazol-1-oxid
5-Chlor-2-(2-chlorphenyl)-2H-benzotriazol-1-oxid
5-Chlor-2-(3-chlorphenyl)-2H-benzotriazol-1-oxid
5-Chlor-2-(2-chlorphenyl)-2H-benzotriazol-1-oxid
5-Chlor-2-(3-chlorphenyl)-2H-benzotriazol-1-oxid
5-Chlor-2-(2,4-dibromphenyl)-2H-benzotriazol-1-oxid
5-Chlor-2-(2,5-dimethylphenyl)-2H-benzotriazol-1-oxid
5-Chlor-2-(4-nitrophenyl)-2H-benzotriazol-1-oxid
5-Chlor-6-nitro-2-phenyl-2H-benzotriazol-1-oxid
2-[4-(4-Chlor-3-nitro-phenylazo)-3-nitrophenyl]-4,6-dinitro-2H-benzotriazol-1-oxid
2-(3-Chlor-4-nitro-phenyl)-4,6-dinitro-2H-benzotriazol-1-oxid
2-(4-Chlor-3-nitrophenyl)-4,6-dinitro-2H-benzotriazol-1-oxid
4-Chlor-6-nitro-2-p-tolyl-2H-benzotriazol-1-oxid
5-Chlor-6-nitro-2-p-tolyl-2H-benzotriazol-1-oxid
6-Chlor-4-nitro-2-p-tolyl-2H-benzotriazol-1-oxid
2-(2-Chlorphenyl)-2H-benzotriazol-1-oxid
2-(3-Chlorphenyl)-2H-benzotriazol-1-oxid
2-(4-Chlorphenyl)-2H-benzotriazol-1-oxid
5-Chlor-2-phenyl-2H-benzotriazol-1-oxid
2-[4-(4-Chlorphenylazo)-3-nitrophenyl]-4,6-dinitro-2H-benzo-tr iazol-1-oxid
2-(2-chlorphenyl)-4,6-dinitro-2H-benzotriazol-1-oxid
2-(3-Chlorphenyl)-4,6-dinitro-2H-benzotriazol-1-oxid
2-(4-Chlorphenyl)-4,6-dinitro-2H-benzotriazol-1-oxid
2-{4-[N'-(3-Chlorphenyl)-hydrazino]-3-nitrophenyl}4,6-di-nitro -2H-benzotriazol-1-oxid
2-{4-[N'-(4-chlorphenyl)-hydrazino]-3-nitrophenyl}4,6-dinitro-2H-benzotriazol-1-oxid
2-(2-chlorphenyl)-6-methyl-2H-benzotriazol-1-oxid
2-(3-chlorphenyl)-6-methyl-2H-benzotriazol-1-oxid
2-(4-chlorphenyl)-6-methyl-2H-benzotriazol-1-oxid
2-(3-Chlorphenyl)-6-nitro-2H-benzotriazol-1-oxid
2-(4-Chlorphenyl)-6-nitro-2H-benzotriazol-1-oxid
2-(4-chlorphenyl)-6-picrylazo-2H-benzotriazol-1-oxid
5-Chlor-2-(2,4,5-trimethylphenyl)-2H-benzotriazol-1-oxid
4,5-Dibrom-6-nitro-2-p-tolyl-2H-benzotriazol-1-oxid
4,5-Dichlor-6-nitro-2-phenyl-2H-benzotriazol-1-oxid
4,5-Dichlor-6-nitro-2-p-tolyl-2H-benzotriazol-1-oxid
4,7-Dichlor-6-nitro-2-p-tolyl-2H-benzotriazol-1-oxid
4,7-Dimethyl-6-nitro-2-phenyl-2H-benzotriazol-1-oxid
2-(2,4-Dimethylphenyl)-4,6-dinitro-benzotriazol-1-oxid
2-(2,5-Dimethylphenyl)-4,6-dinitro-2H-benzotriazol-1-oxid
2-(2,4-Dimethylphenyl)-6-nitro-2H-benzotriazol-1-oxid
2-(2,5-Dimethylphenyl)-6-nitro-2H-benzotriazol-1-oxid
4,6-Dinitro-2-[3-nitro-4-(N'-phenylhydrazino)-phenyl-]-2H-benzotriazol-1-oxid
4,6-Dinitro-2-[4-nitro-4-(N'-phenylhydrazino)-phenyl-]-2H-benzotriazol-1-oxid
4,6-Dinitro-2-phenyl-2H-benzotriazol-1-oxid
2-(2,4-Dinitrophenyl)-4,6-dinitro-2H-benzotriazol-1-oxid
2-(2,4-Dinitrophenyl)-6-nitro-2H-benzotriazol-1-oxid
4,6-Dinitro-2-o-tolyl-2H-benzotriazol-1-oxid
4,6-Dinitro-2-p-tolyl-2H-benzotriazol-1-oxid
4,6-Dinitro-2-(2,4,5-trimethylphenyl)-2H-benzotriazol-1-oxid
2-(4-Methoxyphenyl)-2H-benzotriazol-1-oxid
2-(4-Methoxyphenyl)-6-methyl-2H-benzotriazol-1-oxid
5-Methyl-6-nitro-2-m-tolyl-2H-benzotriazol-1-oxid
5-Methyl-6-nitro-2-o-tolyl-2H-benzotriazol-1-oxid
5-Methyl-6-nitro-2-p-tolyl-2H-benzotriazol-1-oxid
6-Methyl-4-nitro-2-p-tolyl-2H-benzotriazol-1-oxid
6-Methyl-2-phenyl-2H-benzotriazol-1-oxid
4-Methyl-2-m-tolyl-2H-benzotriazol-1-oxid
4-Methyl-2-o-tolyl-2H-benzotriazol-1-oxid
4-Methyl-2-p-tolyl-2H-benzotriazol-1-oxid
6-Methyl-2-m-tolyl-2H-benzotriazol-1-oxid
6-Methyl-2-o-tolyl-2H-benzotriazol-1-oxid
6-Methyl-2-p-tolyl-2H-benzotriazol-1-oxid
2-[1]Naphthyl-4,6-dinitro-2H-benzotriazol-1-oxid
2-[2]Naphthyl-4,6-dinitro-2H-benzotriazol-1-oxid
2-[1]Naphthyl-6-nitro-2H-benzotriazol-1-oxid
2-[2]Naphthyl-6-nitro-2H-benzotriazol-1-oxid
2-(3-Nitrophenyl)-2H-benzotriazol-1-oxid
6-Nitro-2-phenyl-2H-benzotriazol-1-oxid
4-Nitro-2-p-tolyl-2H-benzotriazol-1-oxid
6-Nitro-2-o-tolyl-2H-benzotriazol-1-oxid
6-Nitro-2-p-tolyl-2H-benzotriazol-1-oxid
6-Nitro-2-(2,4,5-trimethylphenyl)-2H-benzotriazol-1-oxid
2-Phenyl-2H-benzotriazol-1-oxid
2-o-Tolyl-2H-benzotriazol-1-oxid
2-p-Tolyl-2H-benzotriazol-1-oxid

Der Mediator kann vorzugsweise ferner ausgewählt sein aus der Gruppe cyclischer N-Hydroxyverbindungen mit mindestens einem ggf. substituierten fünf- oder sechsgliedrigen Ring enthaltend die in Formel V genannte Struktur 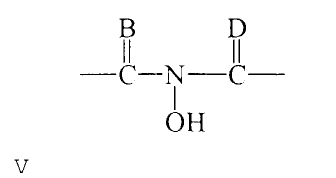 sowie deren Salze, Ether oder Ester, wobei
B und D, gleich oder verschieden sind, und O, S, oder NR¹⁸ bedeuten wobei
oder Salz des Sulfonorests, Sulfamoyl-, Nitro-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeutet,
R¹⁸ Wasserstoff-, Hydroxy-, Formyl-, Carbamoyl-, Sulfonorest, Ester
wobei Carbamoyl-, Sulfamoyl-, Amino- und Phenylreste unsubstituiert oder ein- oder mehrfach mit einem Rest R¹⁹ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R¹⁹ ein- oder mehrfach substituiert sein können wobei
R¹⁹ gleich oder verschieden ist und Hydroxy-, Formyl-, Carboxyrest, Ester oder Salz des Carboxyrests, Carbamoyl-, Sulfonorest, Ester oder Salz des Sulfonorests, Sulfamoyl-, Nitro-, Amino-, Phenyl-, C₁-C₅-Alkyl-, C₁-C₅-Alkoxyrest bedeutet.

Vorzugsweise ist der Mediator ausgewählt aus der Gruppe der Verbindungen der allgemeinen Formel VI, VII, VIII oder IX, 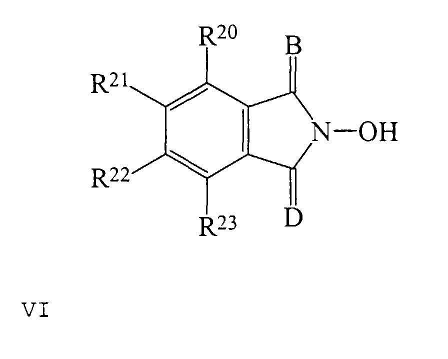 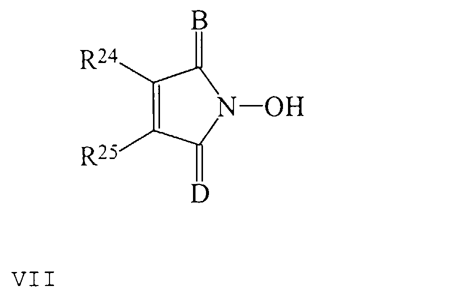 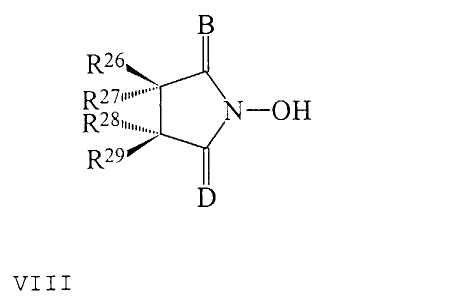 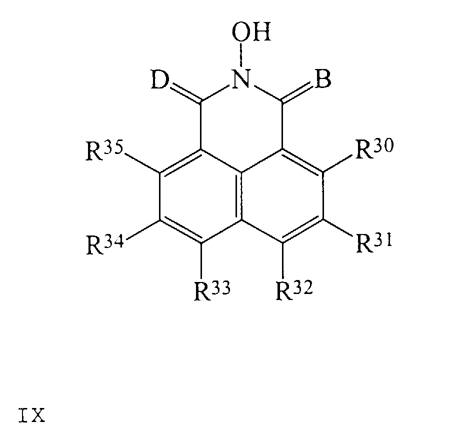
wobei B, D die bereits genannten Bedeutungen haben und die Reste R²⁰-R³⁵ gleich oder verschieden sind und Halogenrest, Carboxyrest, Salz oder Ester eines Carboxyrests oder die für R¹⁸ genannten Bedeutungen haben,
wobei R²⁶ und R²⁷ bzw. R²⁸ und R²⁹ nicht gleichzeitig Hydroxy-oder Aminorest bedeuten dürfen und ggf. je zwei der Substituenten R²⁰-R²³, R²⁴-R²⁵, R²⁶-R²⁹, R³⁰-R³⁵ zu einem Ring -E- verknüpft sein können, wobei -E- eine der folgenden Bedeutungen hat:
   (-CH=CH)-ₙ mit n = 1 bis 3, -CH=CH-CH=N- oder 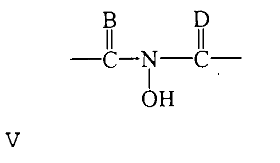
und wobei ggf. die Reste R²⁶-R²⁹ auch untereinander durch ein oder zwei Brückenelemente -F- verbunden sein können, wobei -F-gleich oder verschieden ist und eine der folgende Bedeutungen hat: -O-, -S, -CH₂-, -CR³⁶=CR³⁷- ;
   wobei R³⁶ und R³⁷ gleich oder verschieden sind und die Bedeutung von R²⁰ haben.

Als Mediatoren besonders bevorzugt sind Verbindungen der allgemeinen Formeln VI, VII, VIII oder IX, bei denen B und D O oder S bedeuten.

Beispiele für solche Verbindungen sind N-Hydroxy-phthalimid sowie ggf. substituierte N-Hydroxy-phthalimid-Derivate, N-Hydroxymaleimid sowie ggf. substituierte N-Hydroxymaleimid-Derivate, N-Hydroxy-Naphthalsäureimid sowie ggf. substituierte N-Hydroxy-Naphthalsäureimid-Derivate, N-Hydroxysuccinimid und ggf.substituierte N-Hydroxysuccinimid-Derivate, vorzugsweise solche, bei denen die Reste R²⁶-R²⁹ polycyclisch verbunden sind.

Als Mediator geeignete Verbindungen der Formel VI sind beispielsweise:
N-Hydroxyphthalimid,
3-Amino-N-hydroxyphthalimid,
4-Amino-N-hydroxyphthalimid,
N-Hydroxy-benzol-1,2,4-tricarbonsäureimid,
N,N'-Dihydroxy-pyromellitsäurediimid,
N,N'-Dihydroxy-benzophenon-3,3',4,4'-tetracarbonsäurediimid.

Als Mediator geeignete Verbindungen der Formel VII sind beispielsweise:
N-Hydroxymaleimid,
Pyridin-2,3-dicarbonsäure-N-hydroxyimid.

Als Mediator geeignete Verbindungen der Formel VIII sind beispielsweise:
N-Hydroxysuccinimid,
N-Hydroxyweinsäureimid,
N-Hydroxy-5-norbornen-2,3-dicarbonsäureimid,
exo-N-Hydroxy-7-oxabicyclo[2.2.1]-hept-5-en-2,3-dicarboximid,
N-Hydroxy-cis-cyclohexan-1,2-dicarboximid,
N-Hydroxy-cis-4-cyclohexen-1,2-dicarbonsäureimid.

Als Mediator geeignete Verbindung der Formel IX ist beispielsweise:
N-Hydroxynapthalsäureimid-Natrium-Salz.

Als Mediator geeignete Verbindung mit einem sechsgliedrigen Ring enthaltend die in Formel V genannte Struktur ist beispielsweise:
N-Hydroxyglutarimid.

Die beispielhaft genannten Verbindungen eignen sich auch in Form ihrer Salze oder Ester als Mediator.

Als Mediator ebenfalls geeignet sind Verbindungen ausgewählt aus der Gruppe der N-Aryl-N-hydroxyamide.

Von diesen werden bevorzugt als Mediatoren eingesetzt Verbindungen der allgemeinen Formel X, XI oder XII 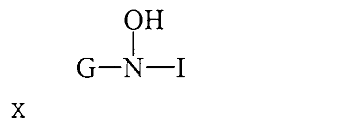 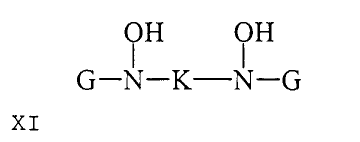 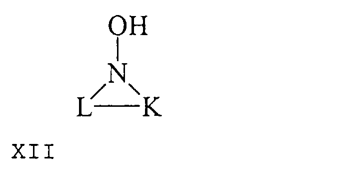 sowie deren Salze, Ether oder Ester, wobei
G einbindiger homo- oder heteroaromatischer ein- oder zweikerniger Rest und
L zweibindiger homo- oder heteroaromatischer ein- oder zweikerniger Rest bedeutet und
wobei diese Aromaten durch einen oder mehrere, gleiche oder verschiedene Reste R³⁸ ausgewählt aus der Gruppe Halogen-, Hydroxy-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests substituiert sein können und
wobei Carbamoyl-, Sulfamoyl-, Amino- und Phenylreste unsubstituiert oder ein- oder mehrfach mit einem Rest R³⁹ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R³⁹ ein- oder mehrfach substituiert sein können, wobei
R³⁹ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxy, C₁ - C₅-Alkylcarbonylrest bedeutet und
je zwei Reste R³⁸ oder R³⁹ paarweise über eine Brücke [-CR⁴⁰R⁴¹-]ₘ mit m gleich 0,1,2, 3 oder 4 verknüpft sein können und
R⁴⁰ und R⁴¹ gleich oder verschieden sind und Carboxyrest, Ester oder Salz des Carboxyrests, Phenyl-, C₁-C₅-Alkyl-, C₁-C₅-Alkoxy, C₁ - C₅-Alkylcarbonylrest bedeuten und eine oder mehrere nicht benachbarte Gruppen [-CR⁴⁰R⁴¹-] durch Sauerstoff, Schwefel oder einen ggf. mit C₁ bis C₅ Alkylrest substituierten Iminorest und zwei benachbarte Gruppen [-CR⁴⁰R⁴¹-] durch eine Gruppe [-CR⁴⁰=CR⁴¹-] ersetzt sein können und
I in amidischer Form vorliegender einbindiger Säurerest von Säuren ausgewählt aus der Gruppe Carbonsäure mit bis zu 20 C-Atomen, Kohlensäure, Halbester der Kohlensäure oder der Carbaminsäure, Sulfonsäure, Phosphonsäure, Phosphorsäure, Monoester der Phosphorsäure, Diester der Phosphorsäure bedeutet und
K in amidischer Form vorliegender zweibindiger Säurerest von Säuren ausgewählt aus der Gruppe Mono- und Dicarbonsäuren mit bis zu 20 C-Atomen, Kohlensäure, Sulfonsäure, Phosphonsäure, Phosphorsäure, Monoester der Phosphorsäure bedeutet.

Als Mediatoren besonders bevorzugt sind Verbindungen der allgemeinen Formel XIII, XIV, XV, XVI oder XVII : 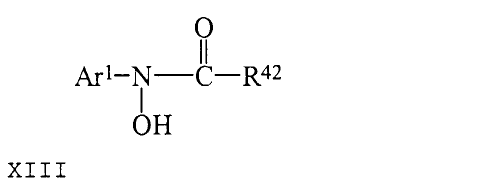 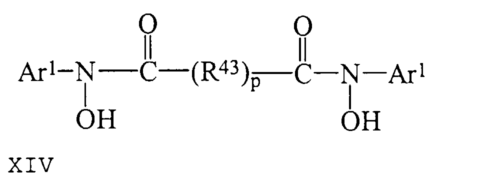 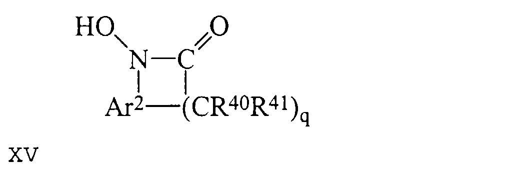 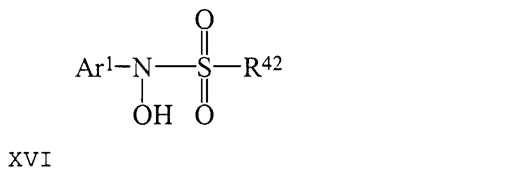 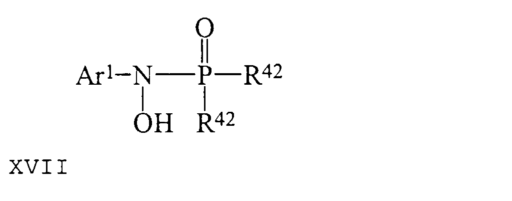 sowie deren Salze, Ether oder Ester, wobei
Ar¹ einbindiger homo- oder heteroaromatischer einkerniger Arylrest und
Ar² zweibindiger homo- oder heteroaromatischer einkerniger Arylrest bedeutet,
die durch eine oder mehrere, gleiche oder verschiedene Reste R⁴⁴ ausgewählt aus der Gruppe Hydroxy-, Cyano-, Carboxyrest,
Ester oder Salz des Carboxyrests, Sulfonorest, Ester oder Salz des Sulfonorests, Nitro-, Nitroso-, Amino-, C₁-C₁₂-Alkyl-, C₁-C₅-Alkoxy-, C₁-C₁₀-Carbonyl-, Carbonyl-C₁-C₆-alkylrest substituiert sein können,
wobei Aminoreste unsubstituiert oder ein- oder mehrfach mit einem Rest R⁴⁵ substituiert sein können und die C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁴⁵ ein- oder mehrfach substituiert sein können,
wobei R⁴⁵ gleich oder verschieden ist und Hydroxy-, Carboxyrest, Ester oder Salz des Carboxyrests, Sulfono-, Nitro-, Amino-, C₁-C₅-Alkyl-, C₁ - C₅-Alkoxy-, C₁ - C₅-Alkylcarbonylrest bedeutet und
je zwei Reste R⁴⁴ paarweise über eine Brücke [-CR⁴⁰R⁴¹-]ₘ mit m gleich 0, 1, 2, 3 oder 4 verknüpft sein können und
R⁴⁰ und R⁴¹ die bereits genannten Bedeutungen haben und eine oder mehrere nicht benachbarte Gruppen [-CR⁴⁰R⁴¹-] durch Sauerstoff, Schwefel oder einen ggf. mit einem C₁ bis C₅ Alkylrest substituierten Iminorest und zwei benachbarte Gruppen [-CR⁴⁰R⁴¹-] durch eine Gruppe [-CR⁴⁰=CR⁴¹-] ersetzt sein können,
R⁴² gleiche oder verschiedene einbindige Reste ausgewählt aus der Gruppe Wasserstoff-, Phenyl-, Aryl-C₁-C₅-alkyl-, C₁-C₁₂-Alkyl-, C₁-C₅-Alkoxy-, C₁-C₁₀-Carbonylrest bedeutet, wobei Phenylreste unsubstituiert oder ein- oder mehrfach mit einem Rest R⁴⁶ substituiert sein können und die Aryl-C₁-C₅-alkyl-, C₁-C₁₂-Alkyl-, C₁-C₅-Alkoxy-, C₁-C₁₀-Carbonyl-Reste -Reste gesättigt oder ungesättigt, verzweigt oder unverzweigt sein können und mit einem Rest R⁴⁶ ein- oder mehrfach substituiert sein können, wobei
R⁴⁶ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxyrest bedeutet und
R⁴³ zweibindige Reste ausgewählt aus der Gruppe ortho-, meta-, para-Phenylen-, Aryl-C₁-C₅-alkyl-, C₁-C₁₂-Alkylen-, C₁-C₅-Alkylendioxyrest bedeutet, wobei Phenylenreste unsubstituiert oder ein- oder mehrfach mit einem Rest R⁴⁶ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁴⁶ ein- oder mehrfach substituiert sein können, wobei
p 0 oder 1 bedeutet und
q eine ganze Zahl von 1 bis 3 bedeutet.

Vorzugsweise bedeutet Ar¹ Phenylrest und
Ar² ortho-Phenylenrest, wobei Ar¹ durch bis zu fünf und Ar² durch bis zu vier gleiche oder verschiedene Reste ausgewählt aus der Gruppe C₁-C₃-Alkyl-, C₁-C₃-Alkylcarbonyl-, Carboxyrest, Ester oder Salz des Carboxyrests, Sulfonorest, Ester oder Salz des Sulfonorests, Hydroxy-, Cyano-, Nitro-, Nitroso- und Aminorest substituiert sein können, wobei Aminoreste mit zwei verschiedenen Resten ausgewählt aus der Gruppe Hydroxy- und C₁-C₃-Alkylcarbonyl substituiert sein können.

Vorzugsweise bedeutet R⁴² einbindiger Rest ausgewählt aus der Gruppe Wasserstoff-, Phenyl-, C₁-C₁₂-Alkyl-, C₁-C₅-Alkoxyrest, wobei die C₁-C₁₂-Alkylreste und C₁-C₅-Alkoxyreste gesättigt oder ungesättigt, verzweigt oder unverzweigt sein können.

Vorzugsweise bedeutet R⁴³ zweibindige Reste ausgewählt aus der Gruppe ortho- oder para-Phenylen-, C₁-C₁₂-Alkylen-, C₁-C₅-Alkylendioxyrest, wobei die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁴⁶ ein- oder mehrfach substituiert sein können.

Vorzugsweise bedeutet R⁴⁶ Carboxyrest, Ester oder Salz des Carboxyrests, Carbamoyl-, Phenyl-, C₁ - C₃-Alkoxyrest.

Beispiele für Verbindungen, die als Mediatoren eingesetzt werden können, sind N-Hydroxyacetanilid, N-Hydroxypivaloylanilid, N-Hydroxyacrylanilid, N-Hydroxybenzoylanilid, N-Hydroxymethylsulfonylanilid, N-Hydroxy-N-phenyl-methylcarbamat, N-Hydroxy-3-oxo-butyrylanilid, N-Hydroxy-4-cyanoacetanilid, N-Hydroxy-4-methoxyacetanilid, N-Hydroxyphenacetin, N-Hydroxy-2,3-dimethylacetanilid, N-Hydroxy-2-methylacetanilid, N-Hydroxy-4-methylacetanilid, l-Hydroxy-3,4-dihydrochinolin-(1H)-2-on, N,N'-Dihydroxy-N,N'-diacetyl-1,3-phenylendiamin, N,N'-Dihydroxy-bernsteinsäuredianilid, N,N'-Dihydroxy-maleinsäuredianilid, N,N'-Dihydroxy-oxalsäuredianilid, N,N'-Dihydroxyphosphorsäuredianilid, N-Acetoxyacetanilid, N-Hydroxymethyloxalylanilid, N-Hydroxymaleinsäuremonoanilid.

Als Mediatoren werden bevorzugt N-Hydroxyacetanilid, N-Hydroxyformanilid, N-Hydroxy-N-phenyl-methylcarbamat, N-Hydroxy-2-methylacetanilid, N-Hydroxy-4-methylacetanilid, 1-Hydroxy-3,4-dihydrochinolin-(1H)-2-on sowie N-Acetoxyacetanilid.

Der Mediator kann ferner ausgewählt sein aus der Gruppe der N-Alkyl-N-Hydroxy-Amide.

Bevorzugt werden dabei als Mediatoren Verbindungen der allgemeinen Formel (XVIII) oder (XIX) 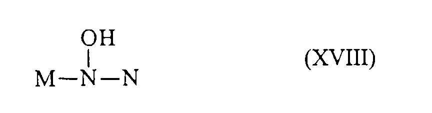 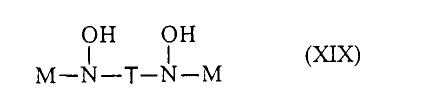 sowie deren Salze, Ether oder Ester eingesetzt, wobei
M gleich oder verschieden ist und einbindiger linearer oder verzweigter oder cyclischer oder polycyclischer gesättigter oder ungesättigter Alkylrest mit 1-24 C-Atomen bedeutet und
wobei dieser Alkylrest durch einen oder mehrere Reste R⁴⁸, die gleich oder verschieden sind und ausgewählt sind aus der Gruppe Hydroxy-, Mercapto-, Formyl-, Carbamoyl-, Carboxy-, Ester oder Salz des Carboxyrests, Sulfonorest, Ester oder Salz des Sulfonorests, Sulfamoyl-, Nitro-, Nitroso-, Amino-, Hydroxylamino-, Phenyl-, C₁-C₅-Alkoxy-, C₁-C₁₀-Carbonyl-, Phospho-, Phosphono-, Phosphonooxyrest, Ester oder Salz des Phosphonooxyrests substituiert sein kann und
wobei Carbamoyl, Sulfamoyl-, Amino-, Hydroxylamino-, Mercapto- und Phenylreste unsubstituiert oder ein- oder mehrfach mit einem Rest R⁴⁸ substituiert sein können und die C₁-C₅-Alkoxy-, C₁-C₁₀-Carbonyl-Reste gesättigt oder ungesättigt, verzweigt oder unverzweigt sein können und mit einem Rest R⁴⁸ ein- oder mehrfach substituiert sein können, wobei
R⁴⁸ gleich oder verschieden ist und Hydroxy-, Formyl-, Cyano-, Carboxyrest, Ester oder Salz des Carboxyrests, Carbamoyl, Sulfono-, Sulfamoyl-, Nitro-, Nitroso-, Amino-, Phenyl-, Benzoyl-, C₁-C₅-Alkyl-, C₁-C₅-Alkoxy-, C₁-C₅-Alkylcarbonylrest bedeutet und
nicht α-ständige Methylengruppen durch Sauerstoff, Schwefel oder einen ggf. einfach substituierten Iminorest ersetzt sein können und
N in amidischer Form vorliegender einbindiger Säurerest von Säuren ausgewählt aus der Gruppe aliphatischer oder ein- oder zweikerniger aromatischer oder ein- oder zweikerniger heteroaromatischer Carbonsäuren mit bis zu 20 C-Atomen, Kohlensäure, Halbester der Kohlensäure oder der Carbaminsäure, Sulfonsäure, Phosphonsäure, Phosphorsäure, Monoester der Phosphorsäure, Diester der Phosphorsäure bedeutet und
T in amidischer Form vorliegender zweibindiger Säurerest von Säuren ausgewählt aus der Gruppe aliphatischer, ein- oder zweikerniger aromatischer oder ein- oder zweikerniger heteroaromatischer Dicarbonsäuren mit bis zu 20 C-Atomen, Kohlensäure, Sulfonsäure, Phosphonsäure, Phosphorsäure, Monoester der Phosphorsäure bedeutet und
wobei Alkylreste der in amidischer Form vorliegenden aliphatischen Säuren N und T linear oder verzweigt und/oder cyclisch und/oder polycyclisch gesättigt oder ungesättigt sein können und 0 - 24 Kohlenstoffatome beinhalten und nicht substituiert sind oder ein- oder mehrfach mit dem Rest R⁴⁷ substituiert sind und

Aryl- und Heteroarylreste der in amidischer Form vorliegenden aromatischen oder heteroaromatischen Säuren N und T durch einen oder mehrere Reste R⁴⁹, die gleich oder verschieden sind und ausgewählt sind aus der Gruppe Hydroxy-, Mercapto-, Formyl-, Cyano-, Carbamoyl-, Carboxy-, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-Alkyl-, C₁-C₁₂-Alkyl-, C₁-C₅-Alkoxy-, C₁-C₁₀-Carbonyl-, Phospho-, Phosphono-, Phosphonooxyrest, Ester oder Salz des Phosphonooxyrests substituiert sein können und
wobei Carbamoyl, Sulfamoyl-, Amino-, Mercapto- und Phenylreste unsubstituiert oder ein- oder mehrfach mit dem Rest R⁴⁸ substituiert sein können und die Aryl-C₁-C₅-Alkyl-, C₁-C₁₂-Alkyl-C₁-C₅-Alkoxy-, C₁-C₁₀-Carbonyl-Reste gesättigt oder ungesättigt, verzweigt oder unverzweigt sein können und ein oder mehrfach mit dem Rest R⁴⁸ substituiert sein können.

Als Mediatoren besonders bevorzugt sind Verbindungen mit den allgemeinen Formel (XX , XXI, XXII oder XXIII): 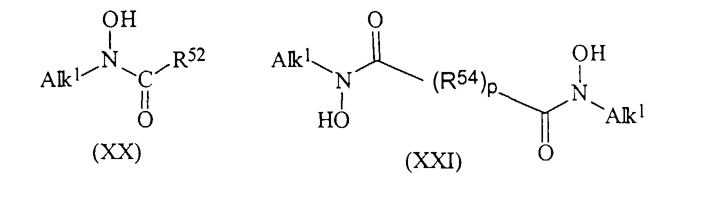 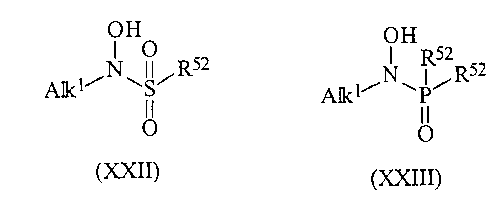 sowie deren Salze, Ether oder Ester, wobei
Alk¹ gleich oder verschieden ist und einbindiger linearer oder verzweigter oder cyclischer oder polycyclischer gesättigter oder ungesättigter Alkylrest mit 1-10 C-Atomen bedeutet, wobei dieser Alkylrest durch einen oder mehrere Reste R⁵⁰, die gleich oder verschieden sind und ausgewählt sind aus der Gruppe Hydroxy-, Formyl-, Carbamoyl-, Carboxy-, Ester oder Salz des Carboxyrests, Sulfonorest, Ester oder Salz des Sulfonorests, Sulfamoyl-, Nitro-, Nitroso-, Amino-, Hydroxylamino-, Phenyl-, C₁-C₅-Alkoxy-, C₁-C₅-Carbonyl-Reste substituiert sein kann und
wobei Carbamoyl, Sulfamoyl-, Amino-, Hydroxylamino- und Phenylreste unsubstituiert oder ein- oder mehrfach mit einem Rest R⁵¹ substituiert sein können und die C₁-C₅-Alkoxy-, C₁-C₁₀-Carbonyl-Reste gesättigt oder ungesättigt, verzweigt oder unverzweigt sein können und mit einem Rest R⁵¹ ein- oder mehrfach substituiert sein können, wobei
R⁵¹ gleich oder verschieden ist und Hydroxy-, Formyl-, Cyano-, Carboxyrest, Ester oder Salz des Carboxyrests, Carbamoyl, Sulfono-, Sulfamoyl-, Nitro-, Amino-, Phenyl-, Benzoyl-, C₁-C₅-Alkyl-, C₁-C₅-Alkoxy-, C₁-C₅-Alkylcarbonylrest bedeutet und
nicht α-ständige Methylengruppen durch Sauerstoff, Schwefel oder einen ggf. einfach substituierten Iminorest ersetzt sein können und
wobei R⁵³ gleiche oder verschiedene einbindige Reste ausgewählt aus der Gruppe Wasserstoff-, Phenyl-, Pyridyl-, Furyl-, Pyrrolyl-, Thienyl-, Aryl-C₁-C₅-Alkyl-, C₁-C₁₂-Alkyl-, C₁-C₁₀-Alkoxy-, C₁-C₁₀-Carbonylrest bedeutet,
wobei Phenyl-, Pyridyl-, Furyl-, Pyrrolyl- und Thienylreste unsubstituiert oder ein- oder mehrfach mit einem Rest R⁷ substituiert sein können und die Aryl-C₁-C₅-alkyl-, C₁-C₁₂-Alkyl-, C₁-C₅-Alkoxy- und C₁-C₁₀-Carbonyl-Reste gesättigt oder ungesättigt, verzweigt oder unverzweigt sein können und mit einem Rest R⁵³ ein- oder mehrfach substituiert sein können und
R⁵³ gleich oder verschieden ist und Hydroxy-, Formyl-, Carboxyrest, Ester oder Salz des Carboxyrests, Carbamoyl-, Sulfono-, Sulfamoyl-, Nitro-, Amino-, Phenyl-, C₁-C₅-Alkyl-, C₁-C₅-Alkoxyrest bedeutet und
R⁵⁴ zweibindige Reste ausgewählt aus der Gruppe Phenylen-, Pyridylen-, Thienylen-, Furylen-, Pyrrolylen-, Aryl-C₁-C₅-alkyl-, C₁-C₁₂-Alkylen-, C₁-C₅-Alkylendioxy-Rest bedeutet, wobei Phenylen-, Pyridylen-, Thienylen-, Furylen-, Pyrrolylen- unsubstituiert oder ein- oder mehrfach mit einem Rest R⁵³ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁵³ ein- oder mehrfach substituiert sein können, wobei
p 0 oder 1 bedeutet.

Als Mediatoren ganz besonders bevorzugt sind Verbindungen der allgemeinen Formel (XX - XXIII), bei denen
Alk¹ gleich oder verschieden ist und einbindiger linearer oder verzweigter oder cyclischer gesättigter oder ungesättigter Alkylrest mit 1-10 C-Atomen bedeutet,
wobei dieser Alkylrest durch einen oder mehrere Reste R⁵⁰, die gleich oder verschieden sind und ausgewählt sind aus der Gruppe Hydroxy-, Carbamoyl-, Carboxy-, Ester oder Salz des Carboxyrests, Sulfonorest, Ester oder Salz des Sulfonorests, Sulfamoyl-, Amino-, Phenyl-, C₁-C₅-Alkoxy-, C₁-C₅-Carbonyl-Reste substituiert sein kann und
wobei Carbamoyl, Sulfamoyl-, Amino- und Phenylreste unsubstituiert oder ein- oder mehrfach mit einem Rest R⁵¹ substituiert sein können und die C₁-C₅-Alkoxy-, C₁-C₁₀-Carbonyl-Reste gesättigt oder ungesättigt, verzweigt oder unverzweigt sein können und mit einem Rest R⁵¹ ein- oder mehrfach substituiert sein können, wobei
R⁵¹ gleich oder verschieden ist und Hydroxy-, Carboxyrest, Ester oder Salz des Carboxyrests, Carbamoyl, Sulfono-, Sulfamoyl-, Nitro-, Amino-, Phenyl-, Benzoyl-, C₁-C₅-Alkyl-, C₁-C₅-Alkoxy-, C₁-C₅-Alkylcarbonylrest bedeutet
   und
wobei R⁵² gleiche oder verschiedene einbindige Reste ausgewählt aus der Gruppe Wasserstoff-, Phenyl-, Furyl-, Aryl-C₁-C₅-Alkyl-, C₁-C₁₂-Alkyl-, C₁-C₁₀-Alkoxy-, C₁-C₁₀-Carbonylrest bedeutet,
wobei Phenyl- und Furylreste unsubstituiert oder ein- oder mehrfach mit einem Rest R⁵³ substituiert sein können und die Aryl-C₁-C₅-alkyl-, C₁-C₁₂-Alkyl-, C₁-C₅-Alkoxy- und C₁-C₁₀-Carbonyl-Reste gesättigt oder ungesättigt, verzweigt oder unverzweigt sein können und mit einem Rest R⁵³ ein- oder mehrfach substituiert sein können,
   wobei
R⁵³ gleich oder verschieden ist und Carboxyrest, Ester oder Salz des Carboxyrests, Carbamoyl-, Phenyl-, C₁-C₅-Alkyl-, C₁-C₅-Alkoxyrest bedeutet und
R⁵⁴ zweibindiger Rest ausgewählt aus der Gruppe Phenylen-, Furylen-, C₁-C₁₂-Alkylen-, C₁-C₅-Alkylendioxy-Rest bedeutet, wobei Phenylen-, Furanylen- unsubstituiert oder ein- oder mehrfach mit einem Rest R⁵³ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁵³ ein- oder mehrfach substituiert sein können, wobei
p 0 oder 1 bedeutet.

Beispiele für Verbindungen, die als Mediatoren eingesetzt werden können, sind
N-Hydroxy-N-methyl-benzoesäureamid, N-Hydroxy-N-methyl-benzolsulfonsäure-amid,
N-Hydroxy-N-methyl-furan-2-carbonsäureamid,
N-Hydroxy-N-methyl-thiophen-2-carbonsäureamid,
N-Hydroxy-N-methyl-thiophen-2-carbonsäureamid, N,N'-Dihydroxy-N,N'-dimethyl-phthalsäurediamid,
N,N'-Dihydroxy-N,N'-dimethyl-isophthalsäurediamid, N,N'-Dihydroxy-N,N'-dimethyl-terephthalsäurediamid,
N,N'-Dihydroxy-N,N'-dimethyl-benzol-1,3-disulfonsäurediamid,
N,N'-Dihydroxy-N,N'-dimethyl-furan-3,4-dicarbonsäurediamid,
N-Hydroxy-N-tert.-butyl-benzoesäureamid,
N-Hydroxy-N-tert.-butyl-benzolsulfonsäureamid,
N-Hydroxy-N-tert.-butyl-p-toluolsulfonsäureamid,
N-Hydroxy-N-tert.-butyl-furan-2-carbonsäureamid,
N-Hydroxy-N-tert.-butyl-thiophen-2-carbonsäureamid,
N,N'-Dihydroxy-N,N'-di-tert.-butyl-phthalsäurediamid,
N,N'-Dihydroxy-N,N'-di-tert.-butyl-isophthalsäurediamid,
N,N'-Dihydroxy-N,N'-di-tert.-butyl-terephthalsäurediamid,
N,N'-Dihydroxy-N,N'-di-tert.-butyl-benzol-1,3-disulfonsäurediamid,
N,N'-Dihydroxy-N,N'-di-tert.-butyl-furan-3,4-dicarbonsäurediamid,
N-Hydroxy-N-cyclohexyl-benzoesäureamid, N-Hydroxy-N-cyclohexyl-benzolsulfonsäureamid,
N-Hydroxy-N-cyclohexyl-p-toluolsulfonsäure-amid,
N-Hydroxy-N-cyclohexyl-furan-2-carbonsäureamid,
N-Hydroxy-N-cyclohexyl-thiophen-2-carbonsäureamid,
N,N'-Dihydroxy-N,N'-dicyclohexyl-phthalsäurediamid,
N,N'-Dihydroxy-N,N'-dicyclohexyl-isophthalsäurediamid,
N,N'-Dihydroxy-N,N'-dicyclohexyl-terephthalsäurediamid,
N,N'-Dihydroxy-N,N'-dicyclohexyl-benzol-1,3-disulfonsäurediamid,
N,N'-Dihydroxy-N,N'-dicyclohexyl-furan-3,4-dicarbonsäure-diamid,
N-Hydroxy-N-isopropyl-benzoesäureamid, N-Hydroxy-N-isopropylbenzol-sulfonsäureamid, N-Hydroxy-N-isopropyl-p-toluolsulfonsäureamid,N-Hydroxy-N-isopropyl-furan-2-carbonsäureamid,
N-Hydroxy-N-isopropyl-thiophen-2-carbon-säureamid, N,N'-Dihydroxy-N,N'-diisopropyl-phthalsäurediamid, N,N'-Dihydroxy-N,N'-diisopropyl-isophthalsäurediamid, N,N'-Dihydroxy-N,N'-diisopropyl-terephthal-säurediamid, N,N'-Dihydroxy-N,N'-diisopropyl-benzol-1,3-disulfonsäurediamid, N,N'-Dihydroxy-N,N'-diisopropyl-furan-3,4-dicarbonsäurediamid, N-Hydroxy-N-methylacetamid, N-Hydroxy-N-tert.-butyl-acetamid,
N-Hydroxy-N-isopropyl-acetamid,
N-Hydroxy-N-cyclohexyl-acetamid,
N-Hydroxy-N-methyl-pivalinsäureamid,
N-Hydroxy-N-isopropyl-pivalinsäureamid, N-Hydroxy-N-methylacrylamid,
N-Hydroxy-N-tert.-butyl-acrylamid, N-Hydroxy-N-isopropyl-acrylamid, N-Hydroxy-N-cyclohexyl-acrylamid, N-Hydroxy-N-methylmethansulfonamid, N-Hydroxy-N-isopropyl-methansulfonamid, N-Hydroxy-N-isopropyl-methylcarbamat,
N-Hydroxy-N-methyl-3-oxo-buttersäureamid, N,N'-Dihydroxy-N,N'-dibenzoyl-ethylendiamin, N,N'-Dihydroxy-N,N'-dimethylbernsteinsäurediamid,
N,N'-Dihydroxy-N,N'-di-tert.-butyl-maleinsäurediamid,
N-Hydroxy-N-tert.-butyl-maleinsäuremonoamid,
N,N'-Dihydroxy-N,N'-di-tert.-butyl-oxalsäurediamid,
N,N'-Dihydroxy-N,N'-di-tert.-butyl-phosphor-säurediamid.

Als Mediatoren werden bevorzugt Verbindungen ausgewählt aus der Gruppe N-Hydroxy-N-methyl-benzoesäureamid, N-Hydroxy-N-methyl-benzolsulfonsäureamid, N-Hydroxy-N-methyl-p-toluolsulfon-säureamid, N-Hydroxy-N-methyl-furan-2-carbonsäureamid,
N,N'-Dihydroxy-N,N'-dimethyl-phthalsäurediamid, N,N'-Dihydroxy-N,N'-dimethyl-terephthalsäurediamid,
N,N'-Dihydroxy-N,N'-dimethyl-benzol-1,3-disulfonsäurediamid,
N-Hydroxy-N-tert.-butyl-benzoesäureamid,
N-Hydroxy-N-tert.-butyl-benzolsulfonsäureamid,
N-Hydroxy-N-tert.-butyl-p-toluolsulfonsäureamid,
N-Hydroxy-N-tert.-butyl-furan-2-carbonsäureamid,
N,N'-Dihydroxy-N,N'-di-tert.-butyl-terephthalsäurediamid, N-Hydroxy-N-isopropyl-benzoesäureamid, N-Hydroxy-N-isopropyl-p-toluolsulfon-säureamid,
N-Hydroxy-N-isopropyl-furan-2-carbonsäureamid, N,N'-Dihydroxy-N,N'-diisopropyl-terephthal-säurediamid, N,N'-Dihydroxy-N,N'-diisopropyl-benzol-1,3-disulfonsäurediamid, N-Hydroxy-N-methyl-acetamid, N-Hydroxy-N-tert.-butyl-acetamid, N-Hydroxy-N-isopropyl-acetamid, N-Hydroxy-N-cyclohexyl-acetamid, N-Hydroxy-N-methyl-pivalinsäureamid,
N-Hydroxy-N-tert.-butyl-acrylamid, N-Hydroxy-N-isopropyl-acrylamid, N-Hydroxy-N-methyl-3-oxo-buttersäureamid, N,N'-Dihydroxy-N,N'-dibenzoyl-ethylendiamin,
N,N'-Dihydroxy-N,N'-di-tert.-butyl-maleinsäurediamid,
N-Hydroxy-N-tert.-butyl-maleinsäuremonoamid,
N,N'-Dihydroxy-N,N'-di-tert.-butyl-oxalsäurediamid.

Der Mediator kann ferner ausgewählt sein aus der Gruppe der Oxime der allgemeinen Formel XXIV oder XXV 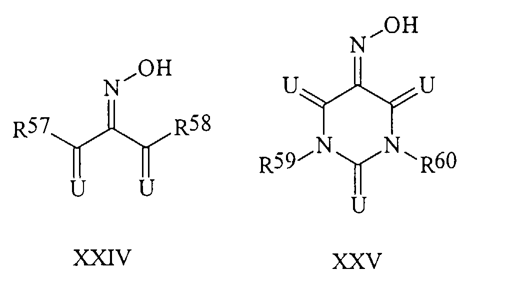 sowie deren Salze, Ether, oder Ester, wobei
U, gleich oder verschieden ist und O, S, oder NR⁵⁵ bedeuten wobei
R⁵⁵ Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeutet,
wobei Carbamoyl-, Sulfamoyl-, Amino- und Phenylreste unsubstituiert oder ein- oder mehrfach mit einem Rest R⁵⁶ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁵⁶ ein- oder mehrfach substituiert sein können, wobei
R⁵⁶ gleich oder verschieden ist und Hydroxy-, Formyl-, Carboxyrest, Ester oder Salz des Carboxyrests, Carbamoyl-, Sulfonorest, Ester oder Salz des Sulfonorests, Sulfamoyl-, Nitro-, Amino-, Phenyl-, C₁-C₅-Alkyl-, C₁-C₅-Alkoxyrest bedeutet und
die Reste R⁵⁷ und R⁵⁸ gleich oder verschieden sind und Halogen-, Carboxyrest, Ester oder Salz des Carboxyrests bedeuten, oder die für R⁵⁵ genannten Bedeutungen haben, oder zu einem Ring [-CR⁶¹R⁶²]ₙ mit n gleich 2, 3 oder 4 verknüpft sind und
R⁵⁹ und R⁶⁰ die für R⁵⁵ genannten Bedeutungen haben und
R⁶¹ und R⁶² gleich oder verschieden sind und Halogen-, Carboxyrest, Ester oder Salz des Carboxyrests bedeuten, oder die für R⁵⁵ genannten Bedeutungen haben.

Als Mediatoren besonders bevorzugt sind Verbindungen der allgemeinen Formel XXIV bei denen U O oder S bedeutet und die übrigen Reste die vorstehend genannten Bedeutungen haben. Ein Beispiel für eine solche Verbindung ist 2-Hydroxyiminomalonsäuredimethylester.

Als Mediatoren weiterhin besonders bevorzugt sind Isonitrosoderivate von cyclischen Ureiden der allgemeinen Formel XXV. Beispiele für solche Verbindungen sind 1-Methylviolursäure, 1,3-Dimethylviolursäure, Thioviolursäure, Alloxan-4,5-dioxim.

Als Mediator insbesondere bevorzugt ist Alloxan-5-oxim Hydrat (Violursäure) und/oder dessen Ester, Ether oder Salze.

Der Mediator kann ferner ausgewählt sein aus der Gruppe vicinal nitrososubstituierter aromatischer Alkohole der allgemeinen Formel XXVI oder XXVII 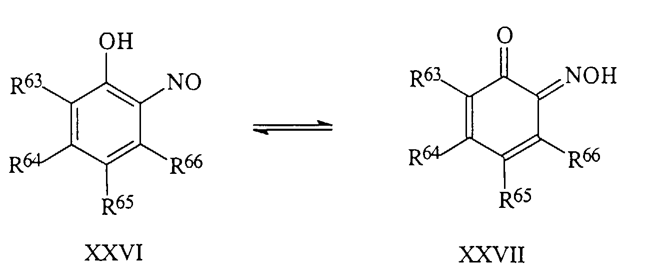 sowie deren Salze, Ether oder Ester, wobei
R⁶³ , R⁶⁴, R⁶⁵ und R⁶⁶ gleich oder verschieden sind und Wasserstoff-, Halogen-, Hydroxy-, Formyl-, Carbamoyl-, Carboxyrest, Ester oder Salz des Carboxyrests, Sulfonorest, Ester oder Salz des Sulfonorests, Sulfamoyl-, Nitro-, Nitroso-, Cyano, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests bedeuten,
wobei Carbamoyl-, Sulfamoyl-, Amino- und Phenylreste unsubstituiert oder ein- oder mehrfach mit einem Rest R⁶⁷ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁶⁷ ein- oder mehrfach substituiert sein können, wobei
R⁶⁷ gleich oder verschieden ist und Hydroxy-, Formyl-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxyrest bedeutet oder
die Reste R⁶³-R⁶⁶ paarweise zu einem Ring [-CR⁶⁸R⁶⁹-]ₘ verknüpft sind, wobei m ganzzahlig ist und einen Wert von 1 bis 4 bedeutet, oder zu einem Ring [-CR⁷⁰=CR⁷¹-]ₙ verknüpft sind, wobei n ganzzahlig ist und einen Wert von 1 bis 3 bedeutet, und
R⁶⁸, R⁶⁹, R⁷⁰ und R⁷¹ gleich oder verschieden sind und die für R⁶³ bis R⁶⁶ genannten Bedeutungen haben.

Unter aromatischen Alkoholen sind vorzugsweise Phenole oder höherkondensierte Derivate des Phenols zu verstehen.

Als Mediatoren bevorzugt sind Verbindungen der allgemeinen Formel XXVI oder XXVII, deren Synthese sich auf die Nitrosierung substituierter Phenole zurückführen läßt. Beispiele für solche Verbindungen sind 2-Nitrosophenol,
3-Methyl-6-nitrosophenol, 2-Methyl-6-nitrosophenol,
4-Methyl-6-nitrosophenol, 3-Ethyl-6-nitrosophenol,
2-Ethyl-6-nitrosophenol, 4-Ethyl-6-nitrosophenol,
4-Isopropyl-6-nitrosophenol, 4-tert.butyl-6-nitrosophenol,
2-Phenyl-6-nitrosophenol, 2-Benzyl-6-nitrosophenol,
4-Benzyl-6-nitrosophenol, 2-Hydroxy-3-nitrosobenzylalkohol,
2-Hydroxy-3-nitrosobenzoesäure,
4-Hydroxy-3-nitrosobenzoesäure, 2-Methoxy-6-nitrosophenol,
3,4-Dimethyl-6-nitrosophenol, 2,4-Dimethyl-6-nitrosophenol,
3,5-Dimethyl-6-nitrosophenol, 2,5-Dimethyl-6-nitrosophenol,
2-Nitrosoresorcin, 4-Nitrosoresorcin, 2-Nitrosoorcin,
2-Nitrosophloroglucin und 4-Nitrosopyrogallol,
4-Nitroso-3-hydroxyanilin, 4-Nitro-2-nitrosophenol.

Als Mediatoren weiterhin bevorzugt sind o-Nitrosoderivate höher kondensierter aromatischer Alkohole. Beispiele für solche Verbindungen sind 2-Nitroso-1-naphthol,
1-Methyl-3-nitroso-2-naphthol und
9-Hydroxy-10-nitroso-phenanthren.

Der Mediator kann ferner ausgewählt sein aus der Gruppe Hydroxypyridine, Aminopyridine, Hydroxychinoline, Aminochinoline, Hydroxyisochinoline, Aminoisochinoline, mit zu den Hydroxy- oder Aminogruppen ortho- oder para-ständigen Nitroso- oder Mercaptosubstituenten, Tautomere der genannten Verbindungen sowie deren Salze, Ether und Ester.

Bevorzugt sind als Mediatoren Verbindungen der allgemeinen Formel (XXVIII), (XXIX) oder (XXX) 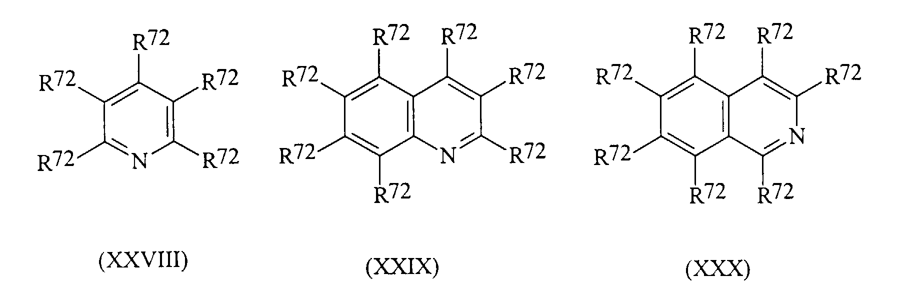 sowie Tautomere, Salze, Ether oder Ester der genannten Verbindungen vorhanden, wobei in den Formeln XXVIII, XXIX und XX zwei zueinander ortho- oder para- ständige Reste R⁷² Hydroxy- und Nitrosorest oder Hydroxy- und Mercaptorest oder Nitrosorest und Aminorest bedeuten
und die übrigen Reste R⁷² gleich oder verschieden sind und ausgewählt sind aus der Gruppe Wasserstoff-, Halogen-, Hydroxy-, Mercapto-, Formyl-, Cyano-, Carbamoyl-, Carboxyrest, Ester und Salz des Carboxyrests, Sulfonorest, Ester und Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester und Salz des Phosphonooxyrests und
wobei Carbamoyl-, Sulfamoyl-, Amino-, Mercapto- und Phenylreste unsubstituiert oder ein- oder mehrfach mit einem Rest R⁷³ substituiert sein können und
die Aryl-C₁-C₅-alkyl-, C₁-C₁₂-Alkyl-, C₁-C₅-Alkoxy-, C₁-C₁₀-Carbonyl-, Carbonyl-C₁-C₆-alkylreste gesättigt oder ungesättigt, verzweigt oder unverzweigt sein können und mit einem Rest R⁷³ ein- oder mehrfach substituiert sein können, wobei
R⁷³ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁-C₅-Alkoxyrest oder C₁-C₅-Alkylcarbonylrest bedeutet und
je zwei Reste R⁷² oder zwei Reste R⁷³ oder R⁷² und R⁷³ paarweise über eine Brücke [-CR⁷⁴R⁷⁵-]ₘmit m gleich 1,2, 3 oder 4 verknüpft sein können und
R³ und R⁴ gleich oder verschieden sind und Carboxyrest, Ester oder Salz des Carboxyrests, Phenyl-, C₁-C₅-Alkyl-, C₁-C₅-Alkoxyrest oder C₁-C₅-Alkylcarbonylrest bedeuten und eine oder mehrere nicht benachbarte Gruppen [-CR⁷⁴R⁷⁵-] durch Sauerstoff, Schwefel oder einen ggf. mit C₁-C₅-Alkyl- substituierten Iminorest und zwei benachbarte Gruppen [-CR⁷⁴R⁷⁵-] durch eine Gruppe [-CR⁷⁴=R⁷⁵-] ersetzt sein können.

Als Mediatoren besonders bevorzugt sind Verbindungen der allgemeinen Formel (XXVIII) oder (XXIX) sowie deren Tautomere, Salze, Ether oder Ester, wobei in den Formeln (XXVIII) und (XXIX) besonders bevorzugt zwei zueinander ortho- ständige Reste R⁷² Hydroxy- und Nitrosorest oder Hydroxy- und Mercaptorest oder Nitrosorest- und Aminorest bedeuten und
die übrigen Reste R⁷² gleich oder verschieden sind und ausgewählt sind aus der Gruppe Wasserstoff-, Hydroxy-, Mercapto-, Formyl-, Carbamoyl-, Carboxyrest, Ester und Salz des Carboxyrests, Sulfonorest, Ester und Salz des Sulfonorests, Sulfamoyl-, Nitro-, Nitroso-, Amino-, Phenyl-, Aryl-C₁-C₅-alkyl-, C₁-C₅-Alkyl-, C₁-C₅-Alkoxy-, C₁-C₅-Carbonyl-, Carbonyl-C₁-C₆-alkyl-, Phospho-, Phosphono-, Phosphono-oxyrest, Ester und Salz des Phosphonooxyrests
   wobei
Carbamoyl-, Sulfamoyl-, Amino-, Mercapto- und Phenylreste unsubstituiert oder ein- oder mehrfach mit einem Rest R⁷³ substituiert sein können und
die Aryl-C₁-C₅-alkyl-, C₁-C₅-Alkyl-, C₁-C₅-Alkoxy-, C₁-C₅-Carbonyl-, Carbonyl-C₁-C₆-alkyl-Reste gesättigt oder ungesättigt, verzweigt oder unverzweigt sein können und mit einem Rest R⁷³ ein- oder mehrfach substituiert sein können, wobei R⁷³ die bereits genannten Bedeutungen hat und
je zwei Reste R⁷³ paarweise über eine Brücke [-CR⁷⁴R⁷⁵-]ₘ mit m gleich 2, 3 oder 4 verknüpft sein können und
R⁷⁴ und R⁷⁵ die bereits genannten Bedeutungen haben und
eine oder mehrere nicht benachbarte Gruppen [-CR⁷⁴R⁷⁵-] durch Sauerstoff oder einen ggf. mit C₁-C₅-Alkyl- substituierten Iminorest ersetzt sein können.

Beispiele für Verbindungen, die als Mediatoren eingesetzt werden können, sind 2,6-Dihydroxy-3-nitrosopyridin,
2,3-Dihydroxy-4-nitrosopyridin,
2,6-Dihydroxy-3-nitrosopyridin-4-carbonsäure,
2,4-Dihydroxy-3-nitrosopyridin, 3-Hydroxy-2-mercaptopyridin,
2-Hydroxy-3-mercaptopyridin, 2,6-Diamino-3-nitrosopyridin,
2,6-Diamino-3-nitroso-pyridin-4-carbonsäure,
2-Hydroxy-3-nitrosopyridin, 3-Hydroxy-2-nitrosopyridin,
2-Mercapto-3-nitrosopyridin, 3-Mercapto-2-nitrosopyridin,
2-Amino-3-nitrosopyridin, 3-Amino-2-nitrosopyridin,
2,4-Dihydroxy-3-nitrosochinolin, 8-Hydroxy-5-nitrosochinolin,
2,3-Dihydroxy-4-nitrosochinolin,
3-Hydroxy-4-nitrosoisochinolin,
4-Hydroxy-3-nitrosoisochinolin, 8-Hydroxy-5-nitrosoisochinolin sowie Tautomere dieser Verbinungen.

Als Mediatoren sind bevorzugt 2,6-Dihydroxy-3-nitrosopyridin,
2,6-Diamino-3-nitrosopyridin,
2,6-Dihydroxy-3-nitrosopyridin-4-carbonsäure,
2,4-Dihydroxy-3-nitrosopyridin, 2-Hydroxy-3-mercaptopyridin,
2-Mercapto-3-pyridinol, 2,4-Dihydroxy-3-nitrosochinolin,
8-Hydroxy-5-nitrosochinolin, 2,3-Dihydroxy-4-nitrosochinolin sowie Tautomere dieser Verbinungen.

Ganz besonders bevorzugte Mediatoren sind N-Hydroxyphthalimid, 1-Hydroxy-1H-benzotriazol, Violursäure, N-Hydroxyacetanilid, Nitrosonaphthol, Nitrosopyridinol sowie deren oben angeführte Derivate.

Am meisten bevorzugt sind 3-Amino-N-hydroxyphthalimid, 4-Amino-N-hydroxyphthalimid, N-Hydroxyphthalimid,
3-Hydroxy-N-hydroxyphthalimid, 3-Methoxy-N-hydroxyphthalimid,
3,4-Dimethoxy-N-hydroxyphthalimid,
4,5-Dimethoxy-N-hydroxyphthalimid,
3,6-Dihydroxy-N-hydroxyphthalimid,
3,6-Dimethoxy-N-hydroxyphthalimid,
3-Methyl-N-hydroxyphthalimid, 4-Methyl-N-hydroxyphthalimid,
3,4-Dimethyl-N-hydroxyphthalimid,
3,5-Dimethyl-N-hydroxyphthalimid,
3,6-Dimethyl-N-hydroxyphthalimid,
3 -Isopropyl-6-methyl-N-hydroxyphthalimid,
3-Nitro-N-hydroxyphthalimid, 4-Nitro-N-hydroxyphthalimid,
1-Hydroxy-1H-benzotriazol, Violursäure und N-Hydroxyacetanilid, 3-Nitrosochinolin-2,4-diol,
2,4-Dihydroxy-3-nitrosopyridin,
2,6-Dihydroxy-3-nitrosopyridin,
2,4-Dinitroso-1,3-dihydroxybenzol,
2-Nitroso-1-naphthol-4-sulfonsäure und
1-Nitroso-2-naphthol-3,6-disulfonsäure.

Die Oxidation wird vorzugsweise in Gegenwart von 0.01 bis 10 Äquivalenten, bevorzugt 0.05 bis 1 Äquivalenten, besonders bevorzugt 0.1 bis 0.5 Äquivalenten eines oder mehrerer der beschriebenen Mediatoren, bevorzugt mit einem oder zwei Mediatoren, besonders bevorzugt mit einem Mediator in Wasser durchgeführt.

Gegebenenfalls geschieht dies unter Zusatz von 1 bis 90 Gewichtsprozenten, bevorzugt 5 bis 30 Gewichtsprozenten eines zumindest teilweise wassermischbaren Lösungsmittels. Vorzugsweise werden 1 bis 3 wassermischbare organische Lösungsmittel als Cosolvens zugesetzt. Beispiele für wassermischbare organische Lösungsmittel sind Ethanol, Methanol, Isopropanol, Ethylenglycol, Ethylenglycolmonoethylether, Ethylenglycolmonomethylether, Aceton, Acetonitril, Acetamid, Tetrahydrofuran, Dioxan, DMSO, DMF, Sulfolan, Essigsäuremethylester, Essigsäureethylester, Ameisensäure, Essigsäure oder Propionsäure oder beliebige Mischungen dieser Lösungsmittel.

Der pH-Wert der Lösung beträgt vorzugsweise 2 bis 8, besonders bevorzugt 4 bis 5.

Die Umsetzungen werden vorzugsweise bei Temperaturen zwischen 5 und 70°C, besonders bevorzugt 35-50°C durchgeführt.

Die Reaktionszeiten betragen vorzugsweise 2 bis 100 Std., bevorzugt 5 bis 50 Std.

Oxidationsmittel sind beispielsweise auch Metalloxide.

Enzyme der Klasse 1.1, die alle Dehydrogenasen, die auf primäre, sekundäre Alkohole und Semiacetale wirken, umfassen und die als Akzeptoren NAD⁺ oder NADP⁺ (Subklasse 1.1.1), Cytochrome (1.1.2), Sauerstoff (O₂) (1.1.3), Disulfide (1.1.4), Chinone (1.1.5) oder die andere Akzeptoren haben (1.1.99).

Aus dieser Klasse sind besonders bevorzugt die Enzyme der Klasse 1.1.5 mit Chinonen als Akzeptoren und die Enzyme der Klasse 1.1.3 mit Sauerstoff als Akzeptor.

Insbesondere bevorzugt in dieser Klasse ist Cellobiose: quinone-1-oxidoreduktase (1.1.5.1).

Weiterhin bevorzugt sind Enzyme der Klasse 1.2. Diese Enzymklasse umfaßt solche Enzyme, die Aldehyde zu den korrespondierenden Säuren oder Oxo-Gruppen oxidieren. Die Akzeptoren können NAD⁺, NADP⁺ (1.2.1), Cytochrome (1.2.2), Sauerstoff (1.2.3), Sulfide (1.2.4), Eisen-Schwefel-Proteine (1.2.5) oder andere Akzeptoren (1.2.99) sein.

Besonders bevorzugt sind hier die Enzyme der Gruppe (1.2.3) mit Sauerstoff als Akzeptor.

Weiterhin bevorzugt sind Enzyme der Klasse 1.3.

In dieser Klasse sind Enzyme zusammengefaßt, die auf CH-CH-Gruppen des Donors wirken.

Die entsprechenden Akzeptoren sind NAD⁺, NADP⁺ (1.3.1), Cytochrome (1.3.2), Sauerstoff (1.3.3), Chinone oder verwandte Verbindungen (1.3.5), Eisen-Schwefel-Proteine (1.3.7) oder andere Akzeptoren (1.3.99).

Besonders bevorzugt ist die Bilirubinoxidase (1.3.3.5).

Hier sind ebenfalls die Enzyme der Klasse (1.3.3) mit Sauerstoff als Akzeptor und (1.3.5) mit Chinonen etc. als Akzeptor besonders bevorzugt.

Weiterhin bevorzugt sind Enzyme der Klasse 1.4, die auf CH-NH₂-Gruppen des Donors wirken.

Die entsprechenden Akzeptoren sind NAD⁺, NADP⁺ (1.4.1), Cytochrome (1.4.2), Sauerstoff (1.4.3), Disulfide (1.4.4), Eisen-Schwefel-Proteine (1.4.7) oder andere Akzeptoren (1.4.99).

Besonders bevorzugt sind auch hier Enzyme der Klasse 1.4.3 mit Sauerstoff als Akzeptor.

Weiterhin bevorzugt sind Enzyme der Klasse 1.5, die auf CH-NH-Gruppen des Donors wirken. Die entsprechenden Akzeptoren sind NAD⁺, NADP⁺ (1.5.1), Sauerstoff (1.5.3), Disulfide (1.5.4), Chinone (1.5.5) oder andere Akzeptoren (1.5.99).

Auch hier sind besonders bevorzugt Enzyme mit Sauerstoff (O₂) (1.5.3) und mit Chinonen (1.5.5) als Akzeptoren.

Weiterhin bevorzugt sind Enzyme der Klasse 1.6, die auf NADH oder NADPH wirken.

Die Akzeptoren sind hier NADP⁺ (1.6.1), Hämproteine (1.6.2), Disulfide (1.6.4), Chinone (1.6.5), NO₂-Gruppen (1.6.6), und ein Flavin (1.6.8) oder einige andere Akzeptoren (1.6.99).

Besonders bevorzugt sind hier Enzyme der Klasse 1.6.5 mit Chinonen als Akzeptoren.

Weiterhin bevorzugt sind Enzyme der Klasse 1.7, die auf andere NO₂-Verbindungen als Donatoren wirken und als Akzeptoren Cytochrome (1.7.2), Sauerstoff (O₂) (1.7.3), Eisen-Schwefel-Proteine (1.7.7) oder andere (1.7.99) haben.

Hier sind besonders bevorzugt die Klasse 1.7.3 mit Sauerstoff als Akzeptor.

Weiterhin bevorzugt sind Enzyme der Klasse 1.8, die auf Schwefelgruppen als Donatoren wirken und als Akzeptoren NAD⁺, NADP⁺ (1.8.1), Cytochrome (1.8.2), Sauerstoff (O₂) (1.8.3), Disulfide (1.8.4), Chinone (1.8.5), Eisen-Schwefel-Proteine (1.8.7) oder andere (1.8.99) haben.

Besonders bevorzugt ist die Klasse 1.8.3 mit Sauerstoff (O₂₎ und (1.8.5) mit Chinonen als Akzeptoren.

Weiterhin bevorzugt sind Enzyme der Klasse 1.9, die auf Hämgruppen als Donatoren wirken und als Akzeptoren Sauerstoff (O₂) (1.9.3), NO₂-Verbindungen (1.9.6) und andere (1.9.99) haben.

Besonders bevorzugt ist hier die Gruppe 1.9.3 mit Sauerstoff (O₂) als Akzeptor (Cytochromoxidasen).

Weiterhin bevorzugt sind Enzyme der Klasse 1.12, die auf Wasserstoff als Donor wirken.

Die Akzeptoren sind NAD⁺ oder NADP⁺ (1.12.1) oder andere (1.12.99).

Desweiteren bevorzugt sind Enzyme der Klasse 1.13 und 1.14 (Oxigenasen).

Weiterhin sind bevorzugte Enzyme die der Klasse 1.15 , die auf Superoxid-Radikale als Akzeptoren wirken.

Besonders bevorzugt ist hier die Superoxid-Dismutase (1.15.1.1).

Weiterhin sind bevorzugt Enzyme der Klasse 1.16.
Als Akzeptoren wirken NAD⁺ oder NADP⁺ (1.16.1) oder Sauerstoff (O₂) (1.16.3).
Besonders bevorzugt sind hier Enzyme der Klasse 1.16.3.1 (Ferroxidase, z.B. Ceruloplasmin).

Weiterhin bevorzugte Enzyme sind diejenigen, die der Gruppe 1.17 (Wirkung auf CH₂-Gruppen, die zu -CHOH- oxidiert werden), 1.18 (Wirkung auf reduziertes Ferredoxin als Donor), 1.19 (Wirkung auf reduziertes Flavodoxin als Donor) und 1.97(andere Oxidoreduktasen) angehören.

Weiterhin besonders bevorzugt sind die Enzyme der Gruppe 1.11. die auf ein Peroxid als Akzeptor wirken. Diese einzige Subklasse (1.11.1) enthält die Peroxidasen.

Besonders bevorzugt sind hier die Cytochrom-c-Peroxidasen (1.11.1.5), Catalase (1.11.1.6), die Peroxydase (1.11.1.7), die Iodid-Peroxidase (1.11.1.8), die Glutathione-Peroxidase (1.11.1.9), die Chlorid-Peroxidase (1.11.1.10), die L-Ascorbat-Peroxidase (1.11.1.11), die Phospholipid-Hydroperoxid-Glutathione-Peroxidase (1.11.1.12), die Mangan-Peroxidase (1.12.1.13), die Diarylpropan-Peroxidase (Ligninase, Lignin-Peroxidase) (1.11.1.14).

Ganz besonders bevorzugt sind Enzyme der Klasse 1.10, die auf Biphenole und verwandten Verbindungen wirken. Sie katalysieren die Oxidation von Biphenolen und Ascorbaten. Als Akzeptoren fungieren NAD⁺, NADP⁺ (1.10.1), Cytochrome (1.10.2), Sauerstoff (1.10.3) oder andere (1.10.99).

Von diesen wiederum sind Enzyme der Klasse 1.10.3 mit Sauerstoff (O₂) als Akzeptor besonders bevorzugt.

Unter den als Oxidationsmittel eingesetzten Metalloxiden werden solche mit einer Löslichkeit unter 1 g/l im Reaktionsmedium bevorzugt.

Bevorzugt sind Wismut(III)oxid, Iridium(III)oxid, Cer(IV)oxid, Cobalt(II)oxid, Cobalt(III)oxid, Eisen(III)oxid, Mangan(I-V)oxid, Zinn(IV)oxid, Niob(V)oxid, Antimon(V)oxid, Indium(II-I)oxid, Quecksilber(II)oxid, Blei(IV)oxid, Silber(I)oxid, Cu(II)oxid, Palladium(II)oxid.

Besonders bevorzugt werden Blei(IV)oxid, Mangan(IV)oxid, Silber(I)oxid, Cu(II)oxid, Palladium(II)oxid.

Die Elektroden der zur Oxidation verwendeten Elektrolysezellen können gleich oder verschieden sein. Sie bestehen vorzugsweise aus Kohlenstoff, Eisen, Blei, Bleidioxid, Kupfer, Nickel, Zink, Cadmium, Quecksilber, Tantal, Titan, Silber, Platin, platiniertem Platin, Palladium, Rhodium oder Gold oder Legierungen aus den genannten Verbindungen.

Besonders bevorzugt sind Edelstähle, Tantal, Titan, Rhodium, Platin oder Gold
Ganz besonders bevorzugt bestehen die Elektroden aus Edelstahl wobei wiederum Edelstählen der Gruppe 1.4xxx (nach DIN 17850) bevorzugt sind.

Die Elektroden können gegebenenfalls durch Bedampfen, Sputtern, Galvanisieren oder ähnliche Verfahren mit anderen Stoffen überzogen worden sein.

Die Oberfläche der Elektroden kann durch geeignete Verfahren vergrößert worden sein, z.B. durch Schleifen, Polieren, Sandstrahlen, Ätzen oder Erodieren.

Mit dem erfindungsgemäßen Verfahren können unter milden Reaktionsbedingungen aromatische und heteroaromatische Aldehyde und Ketone aus den entsprechenden Hydroxyalkylaromaten oder -heteroaromaten hergestellt werden. Die Reaktion wird vorzugsweise in Wasser, gegebenenfalls unter Zusatz eines Cosolvens als Lösungsvermittler durchgeführt und ist dadurch besonders kostengünstig. Die Aufarbeitung der Reaktionslösung erfolgt auf einfache Weise beispielsweise durch Extraktion. Die verwendeteten Mediatoren können katalytisch eingesetzt werden. Besonders rasch reagieren Elektronendonor-substituierte Aromaten.

Die Beispiele erläutern die Erfindung.

### Beispiel 1

Es wurden 22 ml einer Dikaliumhydrogenphosphat/Zitronensäure-Pufferlösung mit pH 4.5 (hergestellt durch Titration einer 0.2 M Kaliumdihydrogenphosphatlösung mit einer 0.1 M Zitronensäurelösung und Verdünnung auf 1/4) bei 45°C mit 269 mg (1.60 mmol) 3,4-Dimethoxybenzylalkohol in 1 ml Ethanol versetzt. Unter Rühren wurden 32.1 mg (0.180 mmol) 3-Amino-N-hydroxyphthalimid zugegeben. Nach ca. 10 min. versetzte man mit 5 ml einer wäßrigen Lösung von 2mg/ml Laccase aus Trametes versicolor (spezifische Aktivität ca. 18 IU / mg, definiert mit ABTS als Substrat). Nach 22 Std. Reaktionszeit wurde die Reaktionslösung mit Chloroform extrahiert und NMR-spektroskopisch untersucht. Ausbeute 92% 3,4-Dimethoxybenzaldehyd.

### Beispiel 2

Eine Oxidation analog zu Beispiel 1 wurde mit 31.9 mg (0.180 mmol) 4-Methyl-N-hydroxyphthalimid als Mediator durchgeführt. HPLC-Analyse ergab eine Ausbeute von 30% 3,4-Dimethoxybenzaldehyd.

### Beispiel 3

Eine Oxidation analog zu Beispiel 1 wurde mit 29.3 mg (0.180 mmol) N-Hydroxyphthalimid als Mediator durchgeführt. HPLC-Analyse ergab eine Ausbeute von 27% 3,4-Dimethoxybenzaldehyd.

### Beispiel 4

Eine Oxidation analog zu Beispiel 1 wurde mit 34.4 mg (0.180 mmol) 3,4-Dimethyl-N-hydroxyphthalimid als Mediator durchgeführt. HPLC-Analyse ergab eine Ausbeute von 32% 3,4-Dimethoxybenzaldehyd.

### Beispiel 5

Eine Oxidation analog zu Beispiel 1 wurde mit 27.2 mg (0.180 mmol) N-Hydroxyacetanilid als Mediator durchgeführt. Ausbeute 51% 3,4-Dimethoxybenzaldehyd.

### Beispiel 6

Wie in Beispiel 1 wurden 173 mg (1.60 mmol) Benzylalkohol mit 32.1 mg (0.180 mmol) 3-Amino-N-hydroxyphthalimid als Mediator umgesetzt. HPLC-Analyse nach 22 Std. Reaktionszeit ergab 22 % Benzaldehyd.

### Beispiel 7

Wie in Beispiel 1 wurden 245 mg (1.60 mmol) 4-Nitrobenzylalkohol mit 32.1 mg (0.180 mmol) 3-Amino-N-hydroxyphthalimid als Mediator umgesetzt. HPLC-Analyse nach 22 Std. Reaktionszeit ergab 41 % 4-Nitrobenzaldehyd.

### Beispiel 8

Wie in Beispiel 1 wurden 196 mg (1.60 mmol) 4-Methylbenzylalkohol mit 32.1 mg (0.180 mmol) 3-Amino-N-hydroxyphthalimid als Mediator umgesetzt. HPLC-Analyse nach 22 Std. Reaktionszeit ergab 36 % 4-Methylbenzaldehyd.

### Beispiel 9

Wie in Beispiel 1 wurden 196 mg (1.60 mmol) 1-Phenylethanol mit 32.1 mg (0.180 mmol) 3-Amino-N-hydroxyphthalimid als Mediator umgesetzt. HPLC-Analyse nach 22 Std. Reaktionszeit ergab 25 % Acetophenon.

### Beispiel 10

Wie in Beispiel 1 wurden 173 mg (1.60 mmol) Benzylalkohol mit 24.3 mg (0.180 mmol) 1-Hydroxy-1H-benzotriazol als Mediator umgesetzt. HPLC-Analyse nach 4 Std. Reaktionszeit ergab 12 % Benzaldehyd.

### Beispiel 11

Wie in Beispiel 1 wurden 245 mg (1.60 mmol) 4-Nitrobenzylalkohol mit 24.3 mg (0.180 mmol) 1-Hydroxy-1H-benzotriazol als Mediator umgesetzt. HPLC-Analyse nach 4 Std. Reaktionszeit ergab 10 % 4-Nitrobenzaldehyd.

### Beispiel 12

Wie in Beispiel 1 wurden 196 mg (1.60 mmol) 4-Methylbenzylalkohol mit 24.3 mg (0.180 mmol) 1-Hydroxy-1H-benzotriazol als Mediator umgesetzt. HPLC-Analyse nach 4 Std. Reaktionszeit ergab 19 % 4-Methylbenzaldehyd.

### Beispiel 13

Wie in Beispiel 1 wurden 196 mg (1.60 mmol) 1-Phenylethanol mit 24.3 mg (0.180 mmol) 1-Hydroxy-1H-benzotriazol als Mediator umgesetzt. HPLC-Analyse nach 4 Std. Reaktionszeit ergab 12 % Acetophenon.

### Beispiel 14

Analog zu Beispiel 1 wurden in 22 ml Pufferlösung 0.40 mmol eines Benzylalkohols (s. Tab.1) und 0.045 mmol Mediator (VIO: Violursäure, HBT: 1-Hydroxy-1H-benzotriazol, NHA: N-Hydroxyacetanilid) in Gegenwart von 2.5 ml einer wäßrigen Lösung von 1mg / ml Laccase umgesetzt. Nach 24 Std. Reaktionszeit wurden die Proben durch HPLC untersucht. Ergebnisse siehe Tab. 1.

**Tabelle 1:**

| Bildung von Benzaldehyden nach 24 Std. | | | |
|---|---|---|---|
| **Edukte** | Mediatoren | | |
| | **ohne [%]** | **HBT [%]** | **NHA [%]** |
| 4-Nitrobenzylalkohol | 0 | 7 | 2 |
| Benzylalkohol | 1 | 22 | 7 |
| 1-Phenylethanol | 0,5 | 10 | 3 |
| 2-Methylbenzylalkohol | 0 | 26 | 9 |
| 4-Methylbenzylalkohol | 0 | 26 | 4 |
| 3,4-Dimethoxybenzylalkohol | 0,5 | 83 | 13 |
| 2,4,6-Trimethylbenzylalkohol | 1 | 5 | 4 |

### Beispiel 15

239 mg (1,0 mmol) Blei(IV)oxid wurden mit 0,40 mmol des jeweiligen Benzylalkohols, 0,05 mmol des Mediators und 25 ml der Pufferlösung (siehe Beispiel 1) versetzt. Das Reaktionsgemisch wurde unter kräftigem Rühren im verschlossenenen Kolben auf 45°C temperiert. Nach 4 und 24 h wurden Proben entnommen. Nach Abfiltrieren des Bleioxids (Sterilfilter) wurde die Produktverteilung durch HPLC-Analyse ermittelt. Ergebnisse in Tab.2.

**Tabelle 2:**

| Oxidation von Benzylalkoholen mit Blei(IV)oxid und verschiedenen Mediatoren (VIO: Violursäure, HBT: 1-Hydroxy-1H-benzotriazol) | | | |
|---|---|---|---|
| Substrat | Mediator | Alkohol | Aldehyd/Keton |
| 3,4-Dimethoxybenzylalkohol | VIO | 96 | 4 |
| 3,4-Dimethoxybenzylalkohol | HBT | 50 | 50 |
| 4-Nitrobenzylalkohol | VIO | 95 | 5 |
| 4-Nitrobenzylalkohol | HBT | 98 | 2 |
| 2-Methylbenzylalkohol | VIO | 93 | 7 |
| 2-Methylbenzylalkohol | HBT | 95 | 5 |
| 1-Phenylethan-1,2-diol | VIO | 96 | 4 |
| 1-Phenylethan-1,2-diol | HBT | 96 | 4 |

### Beispiel 16

0.6 mmol der Benzylalkohole wurden in 40 ml Acetatpuffer (pH 4,5) gelöst und mit Stahlelektroden bei einer Spannung von 40 V und einem Stromfluß von ca. 50 mA 4 Std. bei Raumtemperatur in einem Zweikammersystem elektrolysiert. Die Mediatormenge betrug jeweils 0.15 mmol. Nach 4 Std. wurde das Reaktionsgemisch mit HPLC untersucht. Ergebnisse in Tab.3.

**Tabelle 3:**

| Oxidation von Benzylalkoholen durch elektrochemische Aktivierung von Mediatoren (VIO: Violursäure, HBT: 1-Hydroxy-1H-benzotriazol, NHA: N-hydroxyacetanilid) | | | |
|---|---|---|---|
| Substrat | Mediator | Alkohol | Aldehyd/Keton |
| 3,4-Dimethoxybenzylalkohol | VIO | 95 | 5 |
| 3,4-Dimethoxybenzylalkohol | HBT | 88 | 12 |
| 3,4-Dimethoxybenzylalkohol | NHA | 96 | 4 |
| 4-Nitrobenzylalkohol | VIO | 93 | 7 |
| 4-Nitrobenzylalkohol | HBT | 98 | 2 |
| 4-Nitrobenzylalkohol | NHA | 100 | 0 |
| 2-Methylbenzylalkohol | VIO | 88 | 12 |
| 2-Methylbenzylalkohol | HBT | 97 | 3 |
| 2-Methylbenzylalkohol | NHA | 100 | 0 |
| 4-Methylbenzylalkohol | VIO | 89 | 11 |
| 4-Methylbenzylalkohol | HBT | 96 | 4 |
| 4-Methylbenzylalkohol | NHA | 99 | 1 |
| Benzylalkohol | VIO | 91 | 9 |
| Benzylalkohol | HBT | 96 | 4 |
| Benzylalkohol | NHA | 97 | 3 |
| 1-Phenylethanol | VIO | 94 | 6 |
| 1-Phenylethanol | HBT | 98 | 2 |
| 1-Phenylethanol | NHA | 99 | 1 |
| 2,4,6-Trimethylbenzylalkohol | VIO | 98,5 | 1.5 |
| 2,4,6-Trimethylbenzylalkohol | HBT | 99 | 1 |
| 2,4,6-Trimethylbenzylalkohol | NHA | 100 | 0 |

### Beispiel 17

213 mg (1.59 mmol) 3-Phenyl-2-propen-1-ol in 1.1 ml Ethanol wurden in 22 ml Pufferlösung (siehe Beispiel 1) bei 45°C unter Rühren mit 24.3 mg (0.180 mmol) 1-Hydroxy-1H-benzotriazol versetzt. Nach ca. 10 min. versetzte man mit 5 ml einer wäßrigen Lösung von 2mg/ml Laccase aus Trametes versicolor (spezifische Aktivität ca. 18 IU / mg, definiert mit ABTS als Substrat). Nach 3 Std. unter Luftzutritt entstanden 48% 3-Phenyl-2-propenal (GC-analyse) und es wurden nochmals 24.3 mg HOBT und 5ml Laccaselösung zugesetzt. GC-analyse nach 6 Std. ergab vollständigen Umsatz zu 3-Phenyl-2-propenal.

### Beispiel 18

137 mg (1.59 mmol) 3-Methyl-2-buten-1-ol 1.1 ml Ethanol wurden in 22 ml Pufferlösung (siehe Beispiel 1) bei 45°C unter Rühren mit 24.3 mg (0.180 mmol) 1-Hydroxy-1H-benzotriazol versetzt. Nach ca. 10 min. versetzte man mit 5 ml einer wäßrigen Lösung von 2mg/ml Laccase aus Trametes versicolor (spezifische Aktivität ca. 18 IU / mg, definiert mit ABTS als Substrat). Nach 3 Std. unter Luftzutritt entstanden 52% 3-Methyl-2-butenal neben 24% nichtumgesetztem 3-Methyl-2-butenal (GC-analyse) und es wurden dann nochmals 24.3 mg HOBT und 5ml Laccaselösung zugesetzt. GC- und NMR-analyse ergab nach insgesamt 14 Std. vollständigen Umsatz.

## Patentansprüche

1. Verfahren zur Herstellung von Verbindungen der Formel 1 aus Verbindungen der Formel 2 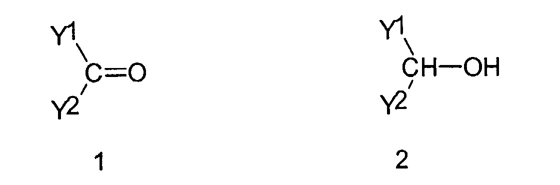 , wobei Y¹ und Y² gleich oder verschieden sein können und Reste mit bis zu 20 C-Atomen und bis zu 6 Ringen bedeuten und mindestens einer der Reste Y¹ oder Y² Wasserstoff, Vinyl, Alkinyl, Aryl oder Heteroaryl bedeutet, und Y¹ und Y² auch Bestandteil eines Ringes oder eines Ringsystems sein können,
mit Hilfe eines Mediators und eines Oxidationsmittels, dadurch gekennzeichnet, daß der Mediator ausgewählt ist aus der Gruppe der aliphatischen, cycloaliphatischen, heterocyclischen oder aromatischen NO- oder NOH-haltigen Verbindungen, und daß das Oxidationsmittel Luft, Sauerstoff, Wasserstoffperoxid, organische Peroxide, Persäuren, Perborate oder Persulfate jeweils in Kombination mit Laccase ist.

2. Verfahren gemäß Anspruch 1, dadurch gekennzeichent, daß Y¹ die Bedeutung hat von aromatischem oder heteroaromatischem Ring oder Ringsystem mit bis zu 6 Ringen und mit bis zu 20 C-Atomen, dessen Ringglieder durch O-, S- oder N-Atome ersetzt sein können oder Anthrachinonylrest,
wobei der aromatische oder heteroaromatische Rest Y¹ ein- bis sechsfach substituiert sein kann,
wobei die Substituenten gleich oder verschieden sein können und die Bedeutung haben von OH, eines linearen, verzweigten oder cyclischen C₃-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe, deren Phenylreste ein- bis fünffach substituiert sein können, wobei die Substituenten gleich oder verschieden sein können und die Bedeutung haben von OH, eines linearen, verzweigten oder cyclischen C₁-C₁₂-Alkylrestes, wobei benachbarte Alkylgruppen über eine Methylengruppe einen 5-, 6-oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe oder
gegebenenfalls ein- bis dreifach substituierter Vinylrest, oder gegebenenfalls substituierter Ethinylrest,
worin die Substituenten gleich oder verschieden sein können und Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können,
oder ein Anthrachinonylrest sein können,
oder worin die Vinylgruppe Bestandteil eines Ringes oder Ringsystems ist und
Y² die Bedeutung hat von Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder
heteroaromatischer Ring oder Ringsystem mit bis zu 6 Ringen und mit bis zu 20 C-Atomen, dessen Ringglieder durch O-, S- oder N-Atome ersetzt sein können, oder Anthrachinonylrest oder gegebenenfalls ein- bis dreifach substituierter Vinylrest, oder gegebenenfalls substituierter Ethinylrest,
worin die Substituenten gleich oder verschieden sein können und Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können, oder ein Anthrachinonylrest sein können,
oder worin die Vinylgruppe Bestandteil eines Ringes oder Ringsystems ist oder
die Reste Y¹ und Y² über eine Methylengruppe oder eine Ethergruppe oder eine gegebenenfalls mit einem linearen,verzweigten oder cyclischen C₁-C₁₂-Alkylrest substituierte Aminogruppe verknüpft sind.

3. Verfahren nach einem der Ansprüche 1 oder 2, dadurch gekennzeichnet, daß als Mediator mindestens eine Verbindung ausgewählt aus der Gruppe N-Hydroxyphthalimid,
1-Hydroxy-1H-benzotriazol, Violursäure, N-Hydroxyacetanilid, Nitrosonaphthol, Nitrosopyridinol eingesetzt wird.

4. Verfahren nach einem oder mehrern der Ansprüche 1 bis 3, dadurch gekennzeichnet, daß als Mediator mindestens eine Verbindung ausgewählt aus der Gruppe 3-Amino-N-hydroxyphthalimid, 4-Amino-N-hydroxyphthalimid, N-Hydroxyphthalimid,
3-Hydroxy-N-hydroxyphthalimid, 3-Methoxy-N-hydroxyphthalimid,
3,4-Dimethoxy-N-hydroxyphthalimid,
4,5-Dimethoxy-N-hydroxyphthalimid,
3,6-Dihydroxy-N-hydroxyphthalimid,
3,6-Dimethoxy-N-hydroxyphthalimid,
3-Methyl-N-hydroxyphthalimid, 4-Methyl-N-hydroxyphthalimid,
3,4-Dimethyl-N-hydroxyphthalimid,
3,5-Dimethyl-N-hydroxyphthalimid,
3,6-Dimethyl-N-hydroxyphthalimid,
3-Isopropyl-6-methyl-N-hydroxyphthalimid,
3-Nitro-N-hydroxyphthalimid, 4-Nitro-N-hydroxyphthalimid,
1-Hydroxy-1H-benzotriazol, Violursäure, N-Hydroxyacetanilid,
3-Nitrosochinolin-2,4-diol, 2,4-Dihydroxy-3-nitrosopyridin,
2,6-Dihydroxy-3-nitrosopyridin,
2,4-Dinitroso-1,3-dihydroxybenzol,
2-Nitroso-1-naphthol-4-sulfonsäure und
1-Nitroso-2-naphthol-3,6-disulfonsäure eingesetzt wird.

5. Verfahren nach Anspruch 1 - 4, dadurch gekennzeichnet, daß als Oxidationsmittel Luft oder Sauerstoff in Kombination mit Laccase eingesetzt wird.

6. Verfahren nach Anspruch 1 - 5, dadurch gekennzeichnet, daß als Oxidationsmittel Metalloxide mit einer Löslichkeit von weniger als lg/1 im Reaktionsmedium eingesetzt werden.

7. Verfahren zur Herstellung von Verbindungen der Formel 1 aus Verbindungen der Formel 2 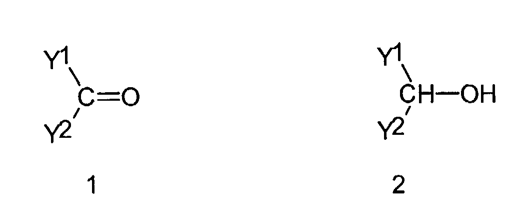 , wobei Y¹ und Y² gleich oder verschieden sein können und Reste mit bis zu 20 C-Atomen und bis zu 6 Ringen bedeuten und mindestens einer der Reste Y¹ oder Y² Wasserstoff, Vinyl, Alkinyl, Aryl oder Heteroaryl bedeutet, und Y¹ und Y² auch Bestandteil eines Ringes oder eines Ringsystems sein können,
mit Hilfe eines Mediators und eines Oxidationsmittels, dadurch gekennzeichnet, daß der Mediator ausgewählt ist aus der Gruppe der Nitrosoderivate von cyclischen Ureiden der Formel XXV und daß Anoden von Elektrolysezellen als Oxidationsmittel eingesetzt werden, wobei Formel XXV sowie deren Salze, Ether, oder Ester, wobei Formel XXV 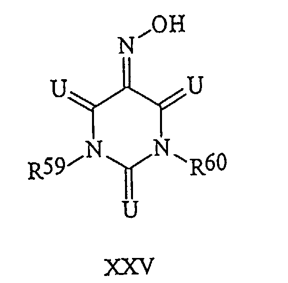 sowie deren Salze, Ehter, oder Ester bedeutet, wobei
U, gleich oder verschieden ist und O, S, oder NR⁵⁵ bedeuten wobei
R⁵⁵ Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeutet,
wobei Carbamoyl-, Sulfamoyl-, Amino- und Phenylreste unsubstituiert oder ein- oder mehrfach mit einem Rest R⁵⁶ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁵⁶ ein- oder mehrfach substituiert sein können, wobei
R⁵⁶ gleich oder verschieden ist und Hydroxy-, Formyl-, Carboxyrest, Ester oder Salz des Carboxyrests, Carbamoyl-, Sulfonorest, Ester oder Salz des Sulfonorests, Sulfamoyl-, Nitro-, Amino-, Phenyl-, C₁-C₅-Alkyl-, C₁-C₅-Alkoxyrest bedeutet und
R⁵⁹ und R⁶⁰ die für R⁵⁵ genannten Bedeutungen haben.

8. Verfahren gemäß Anspruch 7, dadurch gekennzeichent, daß Y¹ die Bedeutung hat von aromatischem oder heteroaromatischem Ring oder Ringsystem mit bis zu 6 Ringen und mit bis zu 20 C-Atomen, dessen Ringglieder durch O-, S- oder N-Atome ersetzt sein können oder Anthrachinonylrest,
wobei der aromatische oder heteroaromatische Rest Y¹ ein- bis sechsfach substituiert sein kann,
wobei die Substituenten gleich oder verschieden sein können und die Bedeutung haben von OH, eines linearen, verzweigten oder cyclischen C₃-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe, deren Phenylreste ein- bis fünffach substituiert sein können, wobei die Substituenten gleich oder verschieden sein können und die Bedeutung haben von OH, eines linearen, verzweigten oder cyclischen C₁-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe oder
gegebenenfalls ein- bis dreifach substituierter Vinylrest, oder gegebenenfalls substituierter Ethinylrest,
worin die Substituenten gleich oder verschieden sein können und Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können, oder ein Anthrachinonylrest sein können,
oder worin die Vinylgruppe Bestandteil eines Ringes oder Ringsystems ist und
Y² die Bedeutung hat von Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder
heteroaromatischer Ring oder Ringsystem mit bis zu 6 Ringen und mit bis zu 20 C-Atomen, dessen Ringglieder durch O-, S- oder N-Atome ersetzt sein können, oder Anthrachinonylrest oder gegebenenfalls ein- bis dreifach substituierter Vinylrest, oder gegebenenfalls substituierter Ethinylrest,
worin die Substituenten gleich oder verschieden sein können und Wasserstoff, linearer, verzweigter oder cyclischer C₁-C₁₂-Alkylrest, bei dem eine oder mehrere Methylengruppen einzeln durch -CHOH, -CO, O-, S-, NH- oder durch einen linearen oder verzweigten C₁-C₁₂-N-Alkylamin-Rest ersetzt sein können oder aromatischer oder heteroaromatischer Ring oder Ringsystem mit bis zu 6 Ringen und mit bis zu 20 C-Atomen, dessen Ringglieder durch O-, S- oder N-Atome ersetzt sein können, oder ein Anthrachinonylrest sein können,
oder worin die Vinylgruppe Bestandteil eines Ringes oder Ringsystems ist oder
die Reste Y¹ und Y² über eine Methylengruppe oder eine Ethergruppe oder eine gegebenenfalls mit einem linearen,verzweigten oder cyclischen C₁-C₁₂-Alkylrest substituierte Aminogruppe verknüpft sind.

9. Verfahren nach Anspruch 1-8, dadurch gekennzeichnet, daß ein bis drei zumindest teilweise wassermischbare Lösungsmittel als Cosolventien zugesetzt werden.

## Claims

1. Process for the preparation of compounds of the formula 1 from compounds of the formula 2 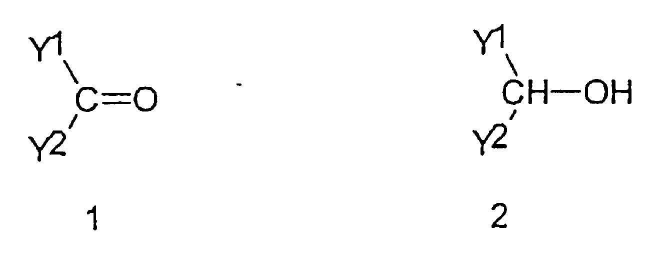 where Y¹ and Y² can be identical or different and are radicals having up to 20 C atoms and up to 6 rings and at least one of the radicals Y¹ or Y² is hydrogen, vinyl, alkynyl, aryl or heteroaryl, and Y¹ and Y² may also be part of a ring or of a ring system, with the aid of a mediator and an oxidant, characterized in that the mediator is selected from the group of the aliphatic, cycloaliphatic, heterocyclic or aromatic NO- or NOH-containing compounds and in that the oxidant is air, oxygen, hydrogen peroxide, organic peroxides, peracids, perborates or persulphates in each case in combination with laccase.

2. Process according to Claim 1, characterized in that Y¹ has the meaning of an aromatic or heteroaromatic ring or ring system having up to 6 rings and up to 20 C atoms, whose ring members can be replaced by O, S or N atoms, or an anthraquinonyl radical,
it being possible for the aromatic or heteroaromatic radical Y¹ to be mono- to hexasubstituted,
it being possible for the substituents to be identical or different and to have the meaning of OH, a linear, branched or cyclic C₃-C₁₂-alkyl radical, it being possible for adjacent alkyl groups to form a 5-, 6- or 7-membered ring via a methylene group, or a linear or branched C₁-C₁₂-oxyalkyl or thioalkyl radical, it being possible for adjacent substituents to form a 5-, 6- or 7-membered ring via a methylene group, a H₂N- or a linear or branched C₁-C₁₂-N-alkylamino, a linear or branched C₁-C₁₂-N,N-dialkylamino group, NC-, O₂N-, halogen, HOOC-, HO₃S-, OHC-, H₂N-COO-, H₂N-CO-, H₂N-CONH-, or a linear, branched or cyclic C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, or a linear or branched C₁-C₁₂-OSO₂-, C₁-C₁₂-NH-SO₂-, or (C₁-C₁₂)₂N-SO₂- group, or a phenyl, diphenylmethyl, phenyl-CH=CH-, phenyl-N=N-, phenyl-N=CH-, phenyl-CH=N-, phenoxy, phenyl-NH-, phenyl-O-CO-, phenyl-CO-, phenyl-NHCO-, phenyl-CONH-, phenyl-NHCONH-, phenyl-OSO₂- or phenyl-NH-SO₂- group whose phenyl radicals can be mono- to pentasubstituted, it being possible for the substituents to be identical or different and to have the meaning of OH, a linear, branched or cyclic C₁-C₁₂-alkyl radical, it being possible for adjacent alkyl groups to form a 5-, 6- or 7-membered ring via a methylene group, or of a linear or branched C₁-C₁₂-oxyalkyl or thioalkyl radical, it being possible for adjacent substituents to form a 5-, 6- or 7-membered ring via a methylene group, a H₂N- or a linear or branched C₁-C₁₂-N-alkylamino, a linear or branched C₁-C₁₂-N,N-dialkylamino group, NC-, O₂N-, halogen, HOOC-, HO₃S-, OHC-, H₂N-COO-, H₂N-CO-, H₂N-CO-NH-, or a linear, branched or cyclic C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, or of a linear or branched C₁-C₁₂-OSO₂-, C₁-C₁₂-NH-SO₂- or (C₁-C₁₂)₂N-SO₂- group, or of a phenyl, diphenylmethyl, phenyl-CH=CH-, phenyl-N=N-, phenyl-N=CH-, phenyl-CH=N-, phenoxy, phenyl-NH-, phenyl-O-CO-, phenyl-CO-, phenyl-NHCO-, phenyl-CONH-, phenyl-NHCONH-, phenyl-OSO₂- or phenyl-NH-SO₂- group or an optionally mono- to trisubstituted vinyl radical, or optionally substituted ethynyl radical, in which the substituents can be identical or different and can be hydrogen, linear, branched or cyclic C₁-C₁₂-alkyl radical where one or more methylene groups can be replaced individually by -CHOH, -CO, O-, S-, NH- or by a linear or branched C₁-C₁₂-N-alkylamine radical, or an aromatic or heteroaromatic ring or ring system having up to 6 rings and up to 20 C atoms, whose ring members can be replaced by O, S or N atoms, or an anthraquinonyl radical, or in which the vinyl group forms part of a ring or ring system, and
Y² has the meaning of hydrogen, linear, branched or cyclic C₁-C₁₂-alkyl radical where one or more methylene groups can be replaced individually by -CHOH, -CO, O-, S-, NH- or by a linear or branched C₁-C₁₂-N-alkylamine radical, or an aromatic or heteroaromatic ring or ring system having up to 6 rings and up to 20 C atoms, whose ring members can be replaced by O, S or N atoms, or anthraquinonyl radical, or an optionally mono- to trisubstituted vinyl radical, or optionally substituted ethynyl radical, in which the substituents can be identical or different and can be hydrogen, linear, branched or cyclic C₁-C₁₂-alkyl radical where one or more methylene groups can be replaced individually by -CHOH, -CO, O-, S-, NH- or by a linear or branched C₁-C₁₂-N-alkylamine radical, or an aromatic or heteroaromatic ring or ring system having up to 6 rings and up to 20 C atoms, whose ring members can be replaced by O, S or N atoms, or an anthraquinonyl radical, or in which the vinyl group forms part of a ring or ring system, or the radicals Y¹ and Y² are linked via a methylene group or an ether group or via an amino group which is optionally substituted by a linear, branched or cyclic C₁-C₁₂-alkyl radical.

3. Process according to either of Claims 1 and 2, characterized in that the mediator employed is at least one compound selected from the group consisting of N-hydroxyphthalimide, 1-hydroxy-1H-benzotriazole, violuric acid, N-hydroxyacetanilide, nitrosonaphthol and nitrosopyridinol.

4. Process according to one or more of Claims 1 to 3, characterized in that the mediator employed is at least one compound selected from the group consisting of 3-amino-N-hydroxyphthalimide, 4-amino-N-hydroxyphthalimide, N-hydroxyphthalimide, 3-hydroxy-N-hydroxyphthalimide, 3-methoxy-N-hydroxyphthalimide, 3,4-dimethoxy-N-hydroxyphthalimide, 4,5-dimethoxy-N-hydroxyphthalimide, 3,6-dihydroxy-N-hydroxyphthalimide, 3,6-dimethoxy-N-hydroxyphthalimide, 3-methyl-N-hydroxyphthalimide, 4-methyl-N-hydroxyphthalimide, 3,4-dimethyl-N-hydroxyphthalimide, 3,5-dimethyl-N-hydroxyphthalimide, 3,6-dimethyl-N-hydroxyphthalimide, 3-isopropyl-6-methyl-N-hydroxyphthalimide, 3-nitro-N-hydroxyphthalimide, 4-nitro-N-hydroxy-phthalimide, 1-hydroxy-1H-benzotriazole, violuric acid, N-hydroxyacetanilide, 3-nitrosoquinoline-2,4-diol, 2,4-dihydroxy-3-nitrosopyridine, 2,6-dihydroxy-3-nitrosopyridine, 2,4-dinitroso-1,3-dihydroxybenzene, 2-nitroso-1-naphthol-4-sulphonic acid and 1-nitroso-2-naphthol-3,6-disulphonic acid.

5. Process according to Claims 1-4, characterized in that the oxidant employed is air or oxygen in combination with laccase.

6. Process according to Claims 1-5, characterized in that the oxidant employed is metal oxides with a solubility of less than 1 g/l in the reaction medium.

7. Process for the preparation of compounds of the formula 1 from compounds of the formula 2 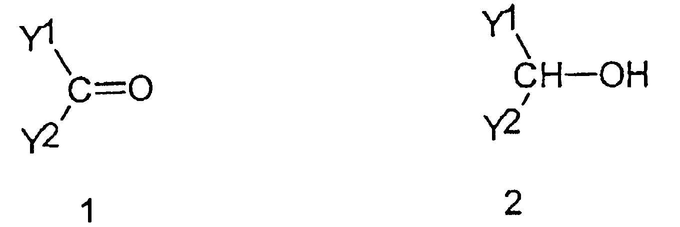 where Y¹ and Y² can be identical or different and are radicals having up to 20 C atoms and up to 6 rings and at least one of the radicals Y¹ or Y² is hydrogen, vinyl, alkynyl, aryl or heteroaryl, and Y¹ and Y² may also be part of a ring or of a ring system,
with the aid of a mediator and an oxidant, characterized in that the mediator is selected from the group of the nitroso derivatives of cyclic ureides of the formula XXV and in that the oxidants used are anodes of electrolytic cells, where formula XXV and its salts, ethers or esters [sic], where formula XXV is 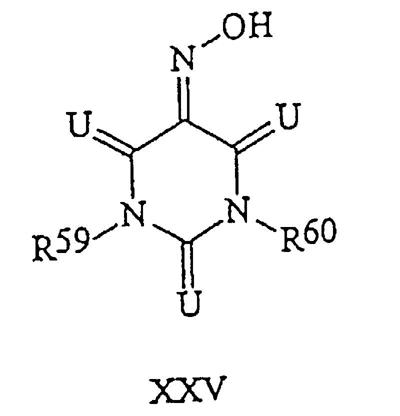 and its salts, ethers or esters, where
U are identical or different and are O, S or NR⁵⁵, where R⁵⁵ is a hydrogen, hydroxyl, formyl, carbamoyl, sulphono radical, an ester or salt of the sulphono radical, a sulphamoyl, nitro, amino, phenyl, aryl-C₁-C₅-alkyl, C₁-C₁₂-alkyl, C₁-C₅-alkoxy, C₁-C₁₀-carbonyl, carbonyl-C₁-C₆-alkyl, phospho, phosphono, phosphonooxy radical, an ester or salt of the phosphonooxy radical,
where the carbamoyl, sulphamoyl, amino and phenyl radicals may be unsubstituted or mono- or polysubstituted by a radical R⁵⁶ and the aryl-C₁-C₅-alkyl, C₁-C₁₂-alkyl, C₁-C₅-alkoxy, C₁-C₁₀-carbonyl, carbonyl-C₁-C₆-alkyl radicals may be saturated or unsaturated, branched or unbranched and may be mono- or polysubstituted by a radical R⁵⁶, where
R⁵⁶ are identical or different and are hydroxyl, formyl, carboxyl radicals, esters or salts of the carboxyl radical, carbamoyl, sulphono radicals, esters or salts of the sulphono radical, sulphamoyl, nitro, amino, phenyl, C₁-C₅-alkyl, C₁-C₅-alkoxy radicals and
R⁵⁹ and R⁶⁰ have the meanings given for R⁵⁵.

8. Process according to Claim 7, characterized in that Y¹ has the meaning of an aromatic or heteroaromatic ring or ring system having up to 6 rings and up to 20 C atoms, whose ring members can be replaced by O, S or N atoms, or an anthraquinonyl radical,
it being possible for the aromatic or heteroaromatic radical Y¹ to be mono- to hexasubstituted,
it being possible for the substituents to be identical or different and to have the meaning of OH, a linear, branched or cyclic C₃-C₁₂-alkyl radical, it being possible for adjacent alkyl groups to form a 5-, 6- or 7-membered ring via a methylene group, or a linear or branched C₁-C₁₂-oxyalkyl or thioalkyl radical, it being possible for adjacent substituents to form a 5-, 6- or 7-membered ring via a methylene group, an H₂N- or a linear or branched C₁-C₁₂-N-alkylamino, a linear or branched C₁-C₁₂-N,N-dialkylamino group, NC-, O₂N-, halogen, HOOC-, HO₃S-, OHC-, H₂N-COO-, H₂N-CO-, H₂N-CONH-, or a linear, branched or cyclic C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, or a linear or branched C₁-C₁₂-OSO₂-, C₁-C₁₂-NH-SO₂-, or (C₁-C₁₂)₂N-SO₂- group, or a phenyl, diphenylmethyl, phenyl-CH=CH-, phenyl-N=N-, phenyl-N=CH-, phenyl-CH=N-, phenoxy, phenyl-NH-, phenyl-O-CO-, phenyl-CO-, phenyl-NHCO-, phenyl-CONH-, phenyl-NHCONH-, phenyl-OSO₂- or phenyl-NH-SO₂- group whose phenyl radicals can be mono- to pentasubstituted, it being possible for the substituents to be identical or different and to have the meaning of OH, a linear, branched or cyclic C₁-C₁₂-alkyl radical, it being possible for adjacent alkyl groups to form a 5-, 6- or 7-membered ring via a methylene group, or of a linear or branched C₁-C₁₂-oxyalkyl or thioalkyl radical, it being possible for adjacent substituents to form a 5-, 6- or 7-membered ring via a methylene group, an H₂N- or a linear or branched C₁-C₁₂-N-alkylamino, a linear or branched C₁-C₁₂-N,N-dialkylamino group, NC-, O₂N-, halogen, HOOC-, HO₃S-, OHC-, H₂N-COO-, H₂N-CO-, H₂N-CO-NH-, or a linear, branched or cyclic C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, or of a linear or branched C₁-C₁₂-OSO₂-, C₁-C₁₂-NH-SO₂- or (C₁-C₁₂)₂N-SO₂- group, or of a phenyl, diphenylmethyl, phenyl-CH=CH-, phenyl-N=N-, phenyl-N=CH-, phenyl-CH=N-, phenoxy, phenyl-NH-, phenyl-O-CO-, phenyl-CO-, phenyl-NHCO-, phenyl-CONH-, phenyl-NHCONH-, phenyl-OSO₂- or phenyl-NH-SO₂- group or an optionally mono- to trisubstituted vinyl radical, or optionally substituted ethynyl radical, in which the substituents can be identical or different and can be hydrogen, linear, branched or cyclic C₁-C₁₂-alkyl radical where one or more methylene groups can be replaced individually by -CHOH, -CO, O-, S-, NH- or by a linear or branched C₁-C₁₂-N-alkylamine radical, or an aromatic or heteroaromatic ring or ring system having up to 6 rings and up to 20 C atoms, whose ring members can be replaced by O, S or N atoms, or an anthraquinonyl radical, or in which the vinyl group forms part of a ring or ring system, and
Y² has the meaning of hydrogen, linear, branched or cyclic C₁-C₁₂-alkyl radical where one or more methylene groups can be replaced individually by -CHOH, -CO, O-, S-, NH- or by a linear or branched C₁-C₁₂-N-alkylamine radical, or an aromatic or heteroaromatic ring or ring system having up to 6 rings and up to 20 C atoms, whose ring members can be replaced by O, S or N atoms, or anthraquinonyl radical, or an optionally mono- to trisubstituted vinyl radical, or optionally substituted ethynyl radical, in which the substituents can be identical or different and can be hydrogen, linear, branched or cyclic C₁-C₁₂-alkyl radical where one or more methylene groups can be replaced individually by -CHOH, -CO, O-, S-, NH- or by a linear or branched C₁-C₁₂-N-alkylamine radical, or an aromatic or heteroaromatic ring or ring system having up to 6 rings and up to 20 C atoms, whose ring members can be replaced by O, S or N atoms, or an anthraquinonyl radical, or in which the vinyl group forms part of a ring or ring system, or the radicals Y¹ and Y² are linked via a methylene group or an ether group or via an amino group which is optionally substituted by a linear, branched or cyclic C₁-C₁₂-alkyl radical.

9. Process according to Claims 1-8, characterized in that one to three solvents which are at least partially miscible with water are added as cosolvents.

## Revendications

1. Procédé de préparation de composés de formule I à partir de composés de formule II 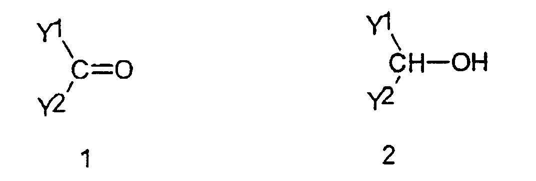 dans lesquelles Y¹ et Y² peuvent être identiques ou différents et représentent des radicaux ayant jusqu'à 20 atomes de carbone et jusqu'à 6 cycles, et au moins l'un des radicaux Y¹ ou Y² représente un hydrogène, un vinyle, un alcynyle, un aryle ou hétéroaryle, et Y¹ et Y² peuvent également être un constituant d'un cycle ou d'un système cyclique,
à l'aide d'un médiateur et d'un oxydant, caractérisé en ce que le médiateur est choisi parmi le groupe constitué de composés à fonction NO ou NOH aliphatiques, cycloaliphatiques, hétérocycliques ou aromatiques, et en ce que l'oxydant est l'air, l'oxygène, le peroxyde d'hydrogène, des peroxydes organiques, des peracides, des perborates ou des persulfates, dans chaque cas en association avec une laccase.

2. Procédé selon la revendication 1, caractérisé en ce que Y¹ représente un cycle ou un système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote ou un radical anthraquinonyle,
où le radical aromatique ou hétéroaromatique Y¹ peut être mono- à hexasubstitué,
où les substituants peuvent être identiques ou différents et représentent OH, un radical alkyle en C₃-C₁₂ linéaire, ramifié ou cyclique, des groupes alkyle adjacents pouvant former un cycle à 5, 6 ou 7 chaînons par l'intermédiaire d'un groupe méthylène, ou un radical oxyalkyle ou thioalkyle en C₁-C₁₂ linéaire ou ramifié, des substituants voisins pouvant former un cycle à 5, 6 ou 7 chaînons par l'intermédiaire d'un groupe méthylène, un groupe H₂N-, ou un groupe C₁-C₁₂-N-alkylamino linéaire ou ramifié, un groupe C₁-C₁₂-N,N-dialkylamino linéaire ou ramifié, un groupe NC-, O₂N-, halogène, HOOC-, HO₃S-, OHC-, H₂N-COO-, H₂N-CO-, H₂N-CO-NH-, ou un groupe C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH- linéaire, ramifié ou cyclique, ou un groupe C₁-C₁₂-OSO₂-, C₁-C₁₂-NH-SO₂- ou (C₁-C₁₂)₂N-SO₂- linéaire ou ramifié, ou un groupe phényle, diphénylméthyle, phényl-CH=CH-, phényl-N=N-, phényl-N=CH-, phényl-CH=N-, phénoxy, phényl-NH-, phényl-O-CO-, phényl-CO-, phényl-NHCO-, phényl-CONH-, phényl-NHCONH-, phényl-OSO₂-ou un groupe phényl-NH-SO₂-, dont les radicaux phényle peuvent être mono- à pentasubstitués, où les substituants peuvent être identiques ou différents et représentent OH, un radical alkyle en C₁-C₁₂ linéaire, ramifié ou cyclique, des groupes alkyle adjacents pouvant former un cycle à 5, 6 ou 7 chaînons par l'intermédiaire d'un groupe méthylène, ou un radical oxyalkyle ou thioalkyle en C₁-C₁₂ linéaire ou ramifié, des substituants voisins pouvant former un cycle à 5, 6 ou 7 chaînons par l'intermédiaire d'un groupe méthylène, un groupe H₂N-, ou un groupe C₁-C₁₂-N-alkylamino linéaire ou ramifié, un groupe C₁-C₁₂-N,N-dialkylamino linéaire ou ramifié, un groupe NC-, O₂N-, halogène, HOOC-, HO₃S-, OHC-, H₂N-COO-, H₂N-CO-, H₂N-CO-NH-, ou un groupe C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH- linéaire, ramifié ou cyclique, ou un groupe C₁-C₁₂-OSO₂-, C₁-C₁₂-NH-SO₂- ou (C₁-C₁₂)₂N-SO₂- linéaire ou ramifié, ou un groupe phényle, diphénylméthyle, phényl-CH=CH-, phényl-N=N-, phényl-N=CH-, phényl-CH=N-, phénoxy-, phényl-NH-, phényl-O-CO-, phényl-CO-, phényl-NHCO-, phényl-CONH-, phényl-NHCONH-, phényl-OSO₂-ou un groupe phényl-NH-SO₂- ou
un radical vinyle éventuellement mono- à trisubstitué, ou un radical éthynyle éventuellement substitué, dans lequel les substituants peuvent être identiques ou différents et peuvent représenter un hydrogène, un radical alkyle en C₁-C₁₂ linéaire, ramifié ou cyclique, dans lequel un ou plusieurs groupes méthylène peuvent être remplacés individuellement par -CHOH, -CO, O-, S-, NH- ou par un radical C₁-C₁₂-N-alkylamine linéaire ou ramifié, ou un cycle ou système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote, ou un radical anthraquinonyle,
ou dans lequel le groupe vinyle est un constituant d'un cycle ou d'un système cyclique, et
Y² représente un hydrogène, un radical alkyle en C₁-C₁₂ linéaire, ramifié ou cyclique, dans lequel un ou plusieurs groupes méthylène peuvent être remplacés individuellement par -CHOH, -CO, O-, S-, NH- ou par un radical C₁-C₁₂-N-alkylamine linéaire ou ramifié, ou un cycle ou système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote, ou un radical anthraquinonyle ou un radical vinyle éventuellement mono- à trisubstitué, ou un radical éthynyle éventuellement substitué, dans lequel les substituants peuvent être identiques ou différents et peuvent représenter un hydrogène, un radical alkyle en C₁-C₁₂ linéaire, ramifié ou cyclique, dans lequel un ou plusieurs groupes méthylène peuvent être remplacés individuellement par -CHOH, -CO, O-, S-, NH- ou par un radical C₁-C₁₂-N-alkylamine linéaire ou ramifié, ou un cycle ou système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote, ou un radical anthraquinonyle,
ou dans lequel le groupe vinyle est un constituant d'un cycle ou d'un système cyclique, ou
les radicaux Y¹ et Y² sont reliés par l'intermédiaire d'un groupe méthylène ou d'un groupe éther ou d'un groupe amino éventuellement substitué par un radical alkyle en C₁-C₁₂ linéaire, ramifié ou cyclique.

3. Procédé selon l'une quelconque des revendications 1 ou 2, caractérisé en ce que l'on utilise, en tant que médiateur, au moins un composé choisi parmi le groupe constitué du N-hydroxyphtalimide, du 1-hydroxy-1H-benzotriazole, de l'acide violurique, du N-hydroxyacétanilide, du nitrosonaphtol et du nitrosopyridinol.

4. Procédé selon l'une quelconque des revendications 1 à 3, caractérisé en ce que l'on utilise, en tant que médiateur, au moins un composé choisi parmi le groupe constitué du 3-amino-N-hydroxyphtalimide, du 4-amino-N-hydroxyphtalimide, du N-hydroxyphtalimide, du 3-hydroxy-N-hydroxyphtalimide, du 3-méthoxy-N-hydroxyphtalimide, du 3,4-diméthoxy-N-hydroxyphtalimide, du 4,5-diméthoxy-N-hydroxyphtalimide, du 3,6-dihydroxy-N-hydroxyphtalimide, du 3,6-diméthoxy-N-hydroxyphtalimide, du 3-méthyl-N-hydroxyphtalimide, du 4-méthyl-N-hydroxyphtalimide, du 3,4-diméthyl-N-hydroxyphtalimide, du 3,5-diméthyl-N-hydroxyphtalimide, du 3,6-diméthyl-N-hydroxyphtalimide, du 3-isopropyl-6-méthyl-N-hydroxyphtalimide, du 3-nitro-N-hydroxyphtalimide, du 4-nitro-N-hydroxyphtalimide, du 1-hydroxy-1H-benzotriazole, de l'acide violurique, du N-hydroxyacétanilide, du 3-nitrosoquinoléine-2,4-diol, de la 2,4-dihydroxy-3-nitrosopyridine, de la 2,6-dihydroxy-3-nitrosopyridine, du 2,4-dinitroso-1,3-dihydroxybenzène, de l'acide 2-nitroso-1-naphtol-4-sulfonique et de l'acide 1-nitroso-2-naphtol-3,6-disulfonique.

5. Procédé selon les revendications 1 à 4, caractérisé en ce que l'on utilise, en tant qu'oxydant, l'air ou l'oxygène en association avec une laccase.

6. Procédé selon les revendications 1 à 5, caractérisé en ce que l'on utilise, en tant qu'oxydant, des oxydes métalliques ayant une solubilité de moins de 1 g/l dans le milieu réactionnel.

7. Procédé de préparation de composés de formule I à partir de composés de formule II 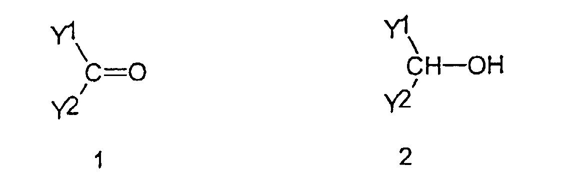 dans lesquelles Y¹ et Y² peuvent être identiques ou différents et représentent des radicaux ayant jusqu'à 20 atomes de carbone et jusqu'à 6 cycles, et au moins l'un des radicaux Y¹ ou Y² représente un hydrogène, un vinyle, un alcynyle, un aryle ou hétéroaryle, et Y¹ et Y² peuvent également être un constituant d'un cycle ou d'un système cyclique,
à l'aide d'un médiateur et d'un oxydant, caractérisé en ce que le médiateur est choisi parmi le groupe constitué de dérivés nitroso d'uréides cycliques de formule XXV et en ce que l'on utilise des anodes de cellules électrolytiques en tant qu'oxydant, où la formule XXV représente 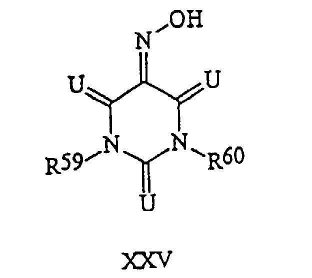 ainsi que ses sels, éthers ou esters, où
U est identique ou différent et représente O, S ou NR⁵⁵, où
R⁵⁵ représente un radical hydrogène, hydroxy, formyle, carbamoyle, sulfono, un ester ou un sel du radical sulfono, un radical sulfamoyle, nitro, amino, phényle, aryl-C₁-C₅-alkyle, alkyle en C₁-C₁₂, alcoxy en C₁-C₅, carbonyle en C₁-C₁₀, carbonyl-C₁-C₆-alkyle, phospho, phosphono, phosphono-oxy, un ester ou un sel du radical phosphono-oxy,
où les radicaux carbamoyle, sulfamoyle, amino et phényle peuvent être non substitués ou mono- ou polysubstitués par un radical R⁵⁶, et les radicaux aryl-C₁-C₅-alkyle, alkyle en C₁-C₁₂, alcoxy en C₁-C₅, carbonyle en C₁-C₁₀, et carbonyl-C₁-C₆-alkyle peuvent être saturés ou insaturés, ramifiés ou non ramifiés et peuvent être mono- ou polysubstitués par un radical R⁵⁶, où
R⁵⁶ est identique ou différent et représente un radical hydroxy, formyle, carboxy, un ester ou un sel du radical carboxy, un radical carbamoyle, sulfono, un ester ou un sel du radical sulfono, un radical sulfamoyle, nitro, amino, phényle, alkyle en C₁-C₅, alcoxy en C₁-C₅, et
R⁵⁹ et R⁶⁰ ont les significations mentionnées pour R⁵⁵.

8. Procédé selon la revendication 7, caractérisé en ce que Y¹ représente un cycle ou un système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote, ou un radical anthraquinonyle, où le radical aromatique ou hétéroaromatique Y¹ peut être mono- à hexasubstitué,
où les substituants peuvent être identiques ou différents et représentent OH, un radical alkyle en C₃-C₁₂ linéaire, ramifié ou cyclique, des groupes alkyle adjacents pouvant former un cycle à 5, 6 ou 7 chaînons par l'intermédiaire d'un groupe méthylène, ou un radical oxyalkyle ou thioalkyle en C₁-C₁₂ linéaire ou ramifié, des substituants voisins pouvant former un cycle à 5, 6 ou 7 chaînons par l'intermédiaire d'un groupe méthylène, un groupe H₂N-, ou un groupe C₁-C₁₂-N-alkylamino linéaire ou ramifié, un groupe C₁-C₁₂-N,N-dialkylamino linéaire ou ramifié, un groupe NC-, O₂N-, halogène, HOOC-, HO₃S-, OHC-, H₂N-COO-, H₂N-CO-, H₂N-CO-NH-, ou un groupe C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH- linéaire, ramifié ou cyclique, ou un groupe C₁-C₁₂-OSO₂-, C₁-C₁₂-NH-SO₂- ou (C₁-C₁₂)₂N-SO₂- linéaire ou ramifié, ou un groupe phényle, diphénylméthyle, phényl-CH=CH-, phényl-N=N-, phényl-N=CH-, phényl-CH=N-, phénoxy, phényl-NH-, phényl-O-CO-, phényl-CO-, phényl-NHCO-, phényl-CONH-, phényl-NHCONH-, phényl-OSO₂- ou un groupe phényl-NH-SO₂-, dont les radicaux phényle peuvent être mono- à pentasubstitués, où les substituants peuvent être identiques ou différents et représentent OH, un radical alkyle en C₁-C₁₂ linéaire, ramifié ou cyclique, des groupes alkyle adjacents pouvant former un cycle à 5, 6 ou 7 chaînons par l'intermédiaire d'un groupe méthylène, ou un radical oxyalkyle ou thioalkyle en C₁-C₁₂ linéaire ou ramifié, des substituants voisins pouvant former un cycle à 5, 6 ou 7 chaînons par l'intermédiaire d'un groupe méthylène, un groupe H₂N-, ou un groupe C₁-C₁₂-N-alkylamino linéaire ou ramifié, un groupe C₁-C₁₂-N,N-dialkylamino linéaire ou ramifié, un groupe NC-, O₂N-, halogène, HOOC-, HO₃S-, OHC-, H₂N-COO-, H₂N-CO-, H₂N-CO-NH-, ou un groupe C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH- linéaire, ramifié ou cyclique, ou un groupe C₁-C₁₂-OSO₂-, C₁-C₁₂-NH-SO₂- ou (C₁-C₁₂)₂N-SO₂- linéaire ou ramifié, ou un groupe phényle, diphénylméthyle, phényl-CH=CH-, phényl-N=N-, phényl-N=CH-, phényl-CH=N-, phénoxy, phényl-NH-, phényl-O-CO-, phényl-CO-, phényl-NHCO-, phényl-CONH-, phényl-NHCONH-, phényl-OSO₂-ou un groupe phényl-NH-SO₂- ou
un radical vinyle éventuellement mono- à trisubstitué, ou un radical éthynyle éventuellement substitué, dans lequel les substituants peuvent être identiques ou différents et peuvent représenter un hydrogène, un radical alkyle en C₁-C₁₂ linéaire, ramifié ou cyclique, dans lequel un ou plusieurs groupes méthylène peuvent être remplacés individuellement par -CHOH, -CO, O-, S-, NH- ou par un radical C₁-C₁₂-N-alkylamine linéaire ou ramifié, ou un cycle ou système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote, ou un radical anthraquinonyle,
ou dans lequel le groupe vinyle est un constituant d'un cycle ou d'un système cyclique, et
Y² représente un hydrogène, un radical alkyle en C₁-C₁₂ linéaire, ramifié ou cyclique, dans lequel un ou plusieurs groupes méthylène peuvent être remplacés individuellement par -CHOH, -CO, O-, S-, NH- ou par un radical C₁-C₁₂-N-alkylamine linéaire ou ramifié, ou un cycle ou système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote, ou un radical anthraquinonyle ou un radical vinyle éventuellement mono- à trisubstitué, ou un radical éthynyle éventuellement substitué, dans lequel les substituants peuvent être identiques ou différents et peuvent représenter un hydrogène, un radical alkyle en C₁-C₁₂ linéaire, ramifié ou cyclique, dans lequel un ou plusieurs groupes méthylène peuvent être remplacés individuellement par -CHOH, -CO, O-, S-, NH- ou par un radical C₁-C₁₂-N-alkylamine linéaire ou ramifié, ou un cycle ou système cyclique aromatique ou hétéroaromatique ayant jusqu'à 6 cycles et jusqu'à 20 atomes de carbone, dont les chaînons du cycle peuvent être remplacés par des atomes d'oxygène, de soufre ou d'azote, ou un radical anthraquinonyle,
ou dans lequel le groupe vinyle est un constituant d'un cycle ou d'un système cyclique, ou
les radicaux Y¹ et Y² sont reliés par l'intermédiaire d'un groupe méthylène ou d'un groupe éther ou d'un groupe amino éventuellement substitué par un radical alkyle en C₁-C₁₂ linéaire, ramifié ou cyclique.

9. Procédé selon les revendications 1 à 8, caractérisé en ce que l'on ajoute un à trois solvants au moins partiellement miscibles à l'eau en tant que co-solvants.
